# EUROPEAN PATENT APPLICATION

(11) **EP 4 461 749 A1**
(43) Date of publication of application: **13.11.2024**
(21) Application number: 23737076.2
(22) Date of filing: 05.01.2023
(51) Int. Cl.: C07K 16/36, A61K 39/395, A61K 39/00, C12N 15/13

(54) **PHARMACEUTICAL COMPOSITION COMPRISING ANTI-FXI/FXIA ANTIBODY AND USE THEREOF**

(30) Priority: 05.01.2022 CN 202210006206; 18.01.2022 CN 202210053441
(71) Applicant: Shanghai Mabgen Biotech Ltd., Shanghai 201206 (CN)
(72) Inventor: SHEN, Lijun, Shanghai 201206 (CN); ZHAO, Zhilong, Shanghai 201206 (CN); LI, Lei, Shanghai 201206 (CN); TANG, Chenxiang, Shanghai 201206 (CN)
(74) Representative: Regimbeau
(86) International application number: PCT/CN2023/070573
(87) International publication number: WO 2023/131213

(57) **Abstract**

A pharmaceutical composition comprising an anti-FXI/FXIa antibody and the use thereof. The specific pharmaceutical composition to which the present disclosure relates comprises an anti-FXI/FXIa antibody or an antigen-binding fragment thereof, and a buffer.

## Description

The present disclosure claims priority to Chinese patent application No. 202210006206.X filed on Jan. 5, 2022 and Chinese patent application No. 202210053441.2 filed on Jan. 18, 2022, both of which are entitled "PHARMACEUTICAL COMPOSITION COMPRISING ANTI-FXIIFXIAANTIBODY AND USE THEREOF" and the contents of which are incorporated herein by reference in their entireties.

### TECHNICAL FIELD

The present disclosure belongs to the field of pharmaceutical formulations and particularly relates to a pharmaceutical composition comprising an anti-FXI/FXIa antibody and an antigen-binding fragment thereof and use thereof as a medicament.

### BACKGROUND

The FXI gene is located in human chromosome 4 and encodes a secreted protein consisting of 607 amino acids. The FXI protein molecule comprises 4 apple domains and 1 catalytic domain, and it is present as a homodimer in human blood. The concentration of FXI protein circulating in human plasma to form a non-covalent complex with HK is about 30 nM (15-45 nM). The human FXI molecule has 88%, 67%, and 58% (two species, three homology data) homology to the monkey and mouse FXI molecules, respectively.

At present, numerous literature and clinical trials have shown that inhibition of the activity of FXI and FXIa to which it converts is effective in reducing thrombosis without creating a visible risk of bleeding. From the clinical results disclosed so far, the anti-FXI/FXIa antibody has similar or better antithrombotic effects with lower bleeding risk and enables safer treatment of patients, as compared to the existing oral anticoagulants (NOACs).

Therapeutic proteins such as human monoclonal antibodies, by their nature, are usually administered by injection as liquid pharmaceutical formulations. However, due to their great molecular weights and complex structures, antibody drugs are prone to degradation, aggregation, unwanted chemical modifications, etc., which make them unstable. It is particularly important to develop stable formulations of antibody drugs that make the antibodies more suitable for administration, keep the antibodies stable during storage and subsequent use, and make the antibodies produce better effects. The present disclosure aims to provide a pharmaceutical composition (formulation) comprising an anti-FXI/FXIa antibody that is sufficiently stable and more suitable for administration.

### SUMMARY

The present disclosure provides a pharmaceutical composition comprising a protein (e.g., an anti-FXI/FXIa antibody or an antigen-binding fragment thereof), a method for preparing the pharmaceutical composition, and a method for treating or preventing a disease using same, or related pharmaceutical use.

In some embodiments, the protein (e.g., the anti-FXI/FXIa antibody or the antigen-binding fragment thereof) in the pharmaceutical composition of the present disclosure has a PI value of about 5.0 to about 6.6, e.g., about 5.2 to about 6.4, about 5.3 to about 6.3, about 5.4 to about 6.2, about 5.5 to about 6.1, about 5.6 to about 6.0, about 5.7 to about 5.9 (e.g., about 5.8), or any range between these point values. In some embodiments, the PI value is obtained by capillary electrophoresis (ProteinSimple, Maurice) under the following conditions:
experiment temperature: 15 °C; focusing time: 1500 V for 1 min; 3000 V for 6 min
method: mixing the reference substance and the test sample (e.g., desalted test sample) and centrifuging at 13000 rpm for 5 min; transferring 80 µL to a Maurice 96-well plate or Maurice iCIEF loading vial, and centrifuging at 13000 rpm for 5 min (for loading through a loading vial) or at 4000 rpm for 5 min (for loading through a 96-well plate).

In some embodiments, the pharmaceutical composition of the present disclosure comprising a protein (e.g., an anti-FXI/FXIa antibody or an antigen-binding fragment thereof) has a conductivity of about 100 mS/cm to about 300 mS/cm, e.g., about 120 mS/cm to about 260 mS/cm, about 130 mS/cm to about 250 mS/cm, about 140 mS/cm to about 240 mS/cm, about 150 mS/cm to about 220 mS/cm, about 170 mS/cm to about 200 mS/cm, about 180 mS/cm to about 190 mS/cm (e.g., about 183.7 mS/cm), or any range between these point values. In some embodiments, the conductivity is measured by a conductivity meter (Mettler; FE38-Standard).

In some embodiments, the pharmaceutical composition of the present disclosure comprising a protein (e.g., an anti-FXI/FXIa antibody or an antigen-binding fragment thereof) has an osmotic pressure of about 250 mOsmol/kg to about 450 mOsmol/kg, e.g., about 270 mOsmol/kg to about 430 mOsmol/kg, about 290 mOsmol/kg to about 410 mOsmol/kg, about 300 mOsmol/kg to about 400 mOsmol/kg, about 310 mOsmol/kg to about 390 mOsmol/kg, about 320 mOsmol/kg to about 380 mOsmol/kg, about 330 mOsmol/kg to about 370 mOsmol/kg, about 340 mOsmol/kg to about 360 mOsmol/kg (e.g., about 350 mOsmol/kg), or any range between these point values. In some embodiments, the osmotic pressure is measured by an osmometer (Loser; OM819.C.), and the detection method is the cryoscopic method.

In some embodiments, the protein (e.g., an anti-FXI/FXIa antibody or an antigen-binding fragment thereof) in the pharmaceutical compositions of the present disclosure has a PI value of about 5.7 to about 5.9. In some embodiments, the pharmaceutical composition of the present disclosure comprising a protein (e.g., an anti-FXI/FXIa antibody or an antigen-binding fragment thereof) has a conductivity of about 180 mS/cm to about 190 mS/cm (e.g., about 183.7 mS/cm) and/or has an osmotic pressure of about 320 mOsmol/kg to about 380 mOsmol/kg.

### Self-buffering system

The present disclosure provides a pharmaceutical composition, which comprises the protein (e.g., an anti-FXI/FXIa antibody or an antigen-binding fragment thereof) according to any one of the above. The pharmaceutical composition is a self-buffering system. The composition has therapeutic or prophylactical activity. In addition, the composition may also have the advantages of good stability and the like.

In some embodiments, the pharmaceutical composition comprises a basic amino acid, such as histidine, arginine, lysine, or any combination thereof. In some embodiments, the basic amino acid (e.g., histidine) forms a self-buffering system with a protein (e.g., an anti-FXI/FXIa antibody or an antigen-binding fragment thereof).

In some embodiments, the histidine in the pharmaceutical composition according to any one of the above has a concentration of about 1 mM to about 100 mM, e.g., about 2 mM to about 80 mM, about 5 mM to about 70 mM, about 10 mM to about 60 mM, about 20 mM to about 50 mM, about 25 mM to about 45 mM, about 30 mM to about 40 mM, about 30 mM to about 100 mM, about 60 mM to about 100 mM, about 30 mM to about 90 mM, or any range between these point values. In some embodiments, the histidine has a concentration of about 5 mM, about 10 mM, about 15 mM, about 20 mM, about 25 mM, about 30 mM, about 31 mM, about 32 mM, about 33 mM, about 34 mM, about 35 mM, about 36 mM, about 37 mM, about 38 mM, about 39 mM, about 40 mM, about 41 mM, about 42 mM, about 43 mM, about 44 mM, about 45 mM, about 46 mM, about 47 mM, about 48 mM, about 49 mM, or about 50 mM, e.g., about 35 mM.

In some embodiments, provided is the pharmaceutical composition according to any one of the above, which comprises a surfactant. In some embodiments, the surfactant is a nonionic surfactant. In some embodiments, the surfactant is selected from the group consisting of poloxamer (e.g., poloxamer 188), polysorbate (e.g., polysorbate 20 (i.e., PS20) or polysorbate 80 (i.e., PS80)), poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocaramide propyl-betaine, linoleinamide propyl-betaine, myristylamide propyl-betaine, palmitamide propyl-betaine, isostearamide propyl-betaine, myristylamide propyl-dimethylamine, palmitamide propyl-dimethylamine, isostearamide propyl-dimethylamine, sodium methyl cocoyl, sodium methyl oleyl taurate, polyethylene glycol, polypropylene glycol, copolymers of ethylene and propylene glycol, and any combinations thereof. In some embodiments, the surfactant is polysorbate or poloxamer. In some embodiments, the surfactant is polysorbate 80, polysorbate 20, or poloxamer 188. In some embodiments, the surfactant is poloxamer 188.

In some embodiments, the surfactant has a concentration of about 0.1 mg/mL to about 10 mg/mL, e.g., about 0.2 mg/mL to about 8 mg/mL, about 0.5 mg/mL to about 6 mg/mL, about 0.8 mg/mL to about 5 mg/mL, about 1.0 mg/mL to about 4 mg/mL, about 1.2 mg/mL to about 3.8 mg/mL, about 1.3 mg/mL to about 3.6 mg/mL, about 1.4 mg/mL to about 3.4 mg/mL, about 1.5 mg/mL to about 3.3 mg/mL, about 1.6 mg/mL to about 3.2 mg/mL, about 1.7 mg/mL to about 3 mg/mL, about 1.8 mg/mL to about 2.8 mg/mL, about 1.9 mg/mL to about 2.5 mg/mL, about 1.8 mg/mL to about 2.4 mg/mL, about 1.9 mg/mL to about 2.2 mg/mL, about 1.8 mg/mL to about 2.2 mg/mL, about 1.9 mg/mL to about 2.1 mg/mL, or any range between these point values. For example, the surfactant has a concentration of about 0.1mg/mL, about 0.2 mg/mL, about 0.3 mg/mL, about 0.4mg/mL, about 0.5 mg/mL, about 0.6 mg/mL, about 0.7 mg/mL, about 0.8 mg/mL, about 0.9 mg/mL, about 1.0 mg/mL, about 1.1 mg/mL, about 1.2 mg/mL, about 1.3 mg/mL, about 1.4 mg/mL, about 1.5 mg/mL, about 1.6 mg/mL, about 1.7 mg/mL, about 1.8 mg/mL, about 1.9 mg/mL, about 2.0 mg/mL, about 2.1 mg/mL, about 2.2 mg/mL, about 2.3 mg/mL, about 2.4 mg/mL, about 2.5 mg/mL, about 2.6 mg/mL, about 2.7 mg/mL, about 2.8 mg/mL, about 2.9 mg/mL, about 3.0 mg/mL, about 3.1 mg/mL, about 3.2 mg/mL, about 3.3 mg/mL, about 3.4 mg/mL, about 3.5 mg/mL, about 3.6 mg/mL, about 3.7 mg/mL, about 3.8 mg/mL, about 3.9 mg/mL, about 4.0 mg/mL, about 4.5 mg/mL, about 5.0 mg/mL, about 5.5 mg/mL, about 6.0 mg/mL, about 6.5 mg/mL, about 7.0 mg/mL, about 7.5 mg/mL, about 8.0 mg/mL, about 8.5 mg/mL, about 9.0 mg/mL, about 9.5 mg/mL, or about 10.0 mg/mL, e.g., about 2 mg/mL.

In some embodiments, provided is the pharmaceutical composition according to any one of the above, which comprises an osmotic pressure regulator. In some embodiments, the osmotic pressure regulator is a sugar (including monosaccharide, disaccharide, trisaccharide, polysaccharide, sugar alcohol, reducing sugar, non-reducing sugar, and the like), an amino acid (including arginine, glycine, cysteine, histidine, and the like), or a salt (sodium chloride, potassium chloride, calcium chloride, and the like). In some embodiments, the osmotic pressure regulator is a sugar, and the sugar is selected from the group consisting of glucose, sucrose, trehalose, lactose, fructose, maltose, dextran, glycerin, erythritol, glycerol, arabitol, sylitol, sorbitol (also known as sorbol), mannitol, melibiose, melezitose, raffinose, mannotriose, stachyose, maltose, lactulose, maltulose, maltitol, lactitol, and iso-maltulose. In some embodiments, the osmotic pressure regulator is selected from the group consisting of one or more of the group consisting of sucrose, trehalose, sorbitol, arginine, proline, glycine, and sodium chloride. In some embodiments, the osmotic pressure regulator is a non-reducing disaccharide. In some embodiments, the osmotic pressure regulator is trehalose or sucrose. In some embodiments, the osmotic pressure regulator is sucrose.

In some embodiments, the osmotic pressure regulator in the pharmaceutical composition according to any one of the above has a concentration of about 1 mg/mL to about 300 mg/mL or about 1 mg/mL to about 400 mg/mL, e.g., about 10 mg/mL to about 400 mg/mL, about 5 mg/mL to about 200 mg/mL, about 10 mg/mL to about 150 mg/mL, about 20 mg/mL to about 140 mg/mL, about 30 mg/mL to about 130 mg/mL, about 40 mg/mL to about 120 mg/mL, 50 mg/mL to about 200 mg/mL, about 50 mg/mL to about 110 mg/mL, about 60 mg/mL to about 100 mg/mL, about 70 mg/mL to about 90 mg/mL (e.g., 80 mg/mL), or any range between these point values. In some embodiments, the osmotic pressure regulator has a concentration of about 10 mg/mL, about 20 mg/mL, about 30 mg/mL, about 40 mg/mL, about 50 mg/mL, about 60 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, or about 200 mg/mL.

In some embodiments, the pharmaceutical composition according to any one of the above has a pH of about 4.0 to about 8.5, e.g., about 5.0 to about 8.0, about 6.0 to about 7.5, about 6.5 to about 7.5, about 6.6 to about 7.4, about 6.7 to about 7.3, about 6.8 to about 7.2, about 6.9 to about 7.1 (e.g., about 7.0), or any range between these point values. Some non-limiting examples of pH include about 4.0, about 4.5, about 5.0, about 6.0, about 6.4, about 6.5, about 6.6, about 6.7, about 6.8, about 6.9, about 7.0, about 7.1, about 7.2, about 7.3, about 7.4, about 7.5, about 7.6, about 7.7, about 7.8, about 7.9, about 8.0, about 8.1, about 8.2, about 8.3, about 8.4, and about 8.5.

Generally, the pH of the pharmaceutical composition obtained by replacing the buffer is almost consistent with the pH of the buffer. Also, it is well known to those skilled in the art that in the process of pharmaceutical formulation, there may sometimes be a pH drift, but the pH drift of the pharmaceutical formulation is generally small (within a range of ±0.3). In some embodiments, the pH drift of the pharmaceutical formulation is within a range of ±0.1.

In some embodiments, the protein (e.g., an anti-FXI/FXIa antibody or an antigen-binding fragment thereof) in the pharmaceutical composition according to any one of the above has a concentration of about 0.1 mg/mL to about 1000 mg/mL, e.g., about 1 mg/mL to about 500 mg/mL, about 1 mg/mL to about 300 mg/mL, about 10 mg/mL to about 400 mg/mL, about 20 mg/mL to about 350 mg/mL, about 30 mg/mL to about 300 mg/mL, about 30 mg/mL to about 200 mg/mL, about 130 mg/mL to about 200 mg/mL, about 40 mg/mL to about 250 mg/mL, about 50 mg/mL to about 200 mg/mL, about 60 mg/mL to about 180 mg/mL, about 50 mg/mL to about 150 mg/mL, about 60 mg/mL to about 150 mg/mL, about 70 mg/mL to about 130 mg/mL, about 80 mg/mL to about 120 mg/mL, about 90 mg/mL to about 110 mg/mL, or any range between these point values. For example, the protein has a concentration of about 1 mg/mL, about 5 mg/mL, about 10 mg/mL, about 15 mg/mL, about 20 mg/mL, about 25 mg/mL, about 30 mg/mL, about 35 mg/mL, about 40 mg/mL, about 45 mg/mL, about 50 mg/mL, about 55 mg/mL, about 60 mg/mL, about 61 mg/mL, about 62 mg/mL, about 63 mg/mL, about 64 mg/mL, about 65 mg/mL, about 66 mg/mL, about 67 mg/mL, about 68 mg/mL, about 69 mg/mL, about 70 mg/mL, about 71 mg/mL, about 72 mg/mL, about 73 mg/mL, about 74 mg/mL, about 75 mg/mL, about 76 mg/mL, about 77 mg/mL, about 78 mg/mL, about 79 mg/mL, about 80 mg/mL, about 81 mg/mL, about 82 mg/mL, about 83 mg/mL, about 84 mg/mL, about 85 mg/mL, about 86 mg/mL, about 87 mg/mL, about 88 mg/mL, about 89 mg/mL, about 90 mg/mL, about 91 mg/mL, about 92 mg/mL, about 93 mg/mL, about 94 mg/mL, about 95 mg/mL, about 96 mg/mL, about 97 mg/mL, about 98 mg/mL, about 99 mg/mL, about 100 mg/mL, about 101 mg/mL, about 102 mg/mL, about 103 mg/mL, about 104 mg/mL, about 105 mg/mL, about 106 mg/mL, about 107 mg/mL, about 108 mg/mL, about 109 mg/mL, about 110 mg/mL, about 111 mg/mL, about 112 mg/mL, about 113 mg/mL, about 114 mg/mL, about 115 mg/mL, about 116 mg/mL, about 117 mg/mL, about 118 mg/mL, about 119 mg/mL, about 120 mg/mL, about 121 mg/mL, about 122 mg/mL, about 123 mg/mL, about 124 mg/mL, about 125 mg/mL, about 126 mg/mL, about 127 mg/mL, about 128 mg/mL, about 129 mg/mL, about 130 mg/mL, about 131 mg/mL, about 132 mg/mL, about 133 mg/mL, about 134 mg/mL, about 135 mg/mL, about 140 mg/mL, about 145 mg/mL, about 150 mg/mL, about 155 mg/mL, about 160 mg/mL, about 165 mg/mL, about 170 mg/mL, about 175 mg/mL, about 180 mg/mL, about 185 mg/mL, about 190 mg/mL, about 195 mg/mL, or about 200 mg/mL.

In some embodiments, the pharmaceutical composition according to any one of the above comprises any one of a)-g):
a) about 1 mg/mL to about 500 mg/mL protein (e.g., an anti-FXI/FXIa antibody or an antigen-binding fragment thereof) and about 1 mM to about 100 mM histidine, the pharmaceutical composition having a pH of about 4.0 to about 8.5; optionally, the pharmaceutical composition may further comprise about 1 mg/mL to about 500 mg/mL sucrose or trehalose and/or about 0.1 mg/mL to about 10 mg/mL poloxamer 188 or polysorbate.
b) about 10 mg/mL to about 300 mg/mL protein (e.g., an anti-FXI/FXIa antibody or an antigen-binding fragment thereof) and about 5 mM to about 80 mM histidine, the pharmaceutical composition having a pH of about 6.0 to about 8.0; optionally, the pharmaceutical composition may further comprise about 10 mg/mL to about 400 mg/mL sucrose or trehalose and/or about 0.5 mg/mL to about 8.0 mg/mL poloxamer 188 or polysorbate.
c) about 30 mg/mL to about 200 mg/mL protein (e.g., an anti-FXI/FXIa antibody or an antigen-binding fragment thereof) and about 10 mM to about 60 mM, about 20 mM to about 50 mM, or about 30 mM to about 40 mM (e.g., about 35 mM) histidine;
d) about 50 mg/mL to about 150 mg/mL protein (e.g., an anti-FXI/FXIa antibody or an antigen-binding fragment thereof) and about 10 mM to about 60 mM, about 20 mM to about 50 mM, or about 30 mM to about 40 mM (e.g., about 35 mM) histidine;
e) about 70 mg/mL to about 130 mg/mL protein (e.g., an anti-FXI/FXIa antibody or an antigen-binding fragment thereof) and about 10 mM to about 60 mM, about 20 mM to about 50 mM, or about 30 mM to about 40 mM (e.g., about 35 mM) histidine;
h) about 130 mg/mL to about 200 mg/mL protein (e.g., an anti-FXI/FXIa antibody or an antigen-binding fragment thereof) and about 30 mM to about 100 mM, about 60 mM to about 100 mM, or about 60 mM to about 90 mM histidine;
I) about 30 mg/mL to about 200 mg/mL protein (e.g., an anti-FXI/FXIa antibody or an antigen-binding fragment thereof) and about 30 mM to about 100 mM, about 60 mM to about 100 mM, or about 60 mM to about 90 mM histidine;
   wherein, the pharmaceutical composition in c)-e) and h)-I) has a pH of about 6.5 to about 7.5 (e.g., about 6.7 to about 7.3, e.g., about 7.0);
   optionally, the pharmaceutical compositions in c)-e) and h)-I) further comprises sucrose or trehalose and poloxamer 188 or polysorbate (e.g., polysorbate 80 or polysorbate 20);
   in some embodiments, the pharmaceutical composition in c)-e) and h)-I) comprises sucrose having a concentration of about 40 mg/mL to about 300 mg/mL, about 50 mg/mL to about 200 mg/mL, about 60 mg/mL to about 150 mg/mL, or about 70 mg/mL to about 120 mg/mL (e.g., about 70 mg/mL, about 80 mg/mL, about 90 mg/mL, about 100 mg/mL, about 110 mg/mL, or about 120 mg/mL);
   in some embodiments, the pharmaceutical compositions in c)-e) and h) -I) comprises poloxamer 188 having a concentration of about 1.5 mg/mL to about 6 mg/mL, about 1.5 mg/mL to about 5.0 mg/mL, about 1.8 mg/mL to about 3.5 mg/mL, or about 1.8 mg/mL to about 2.8 mg/mL (e.g., about 1.8 mg/mL, about 1.9 mg/mL, about 2.0 mg/mL, about 2.1 mg/mL, about 2.2 mg/mL, about 2.3 mg/mL, about 2.4 mg/mL, about 2.5 mg/mL, about 2.6 mg/mL, about 2.7 mg/mL, or about 2.8 mg/mL);
f) about 70 mg/mL to about 130 mg/mL protein (e.g., an anti-FXI/FXIa antibody), about 30 mM to about 40 mM (e.g., about 35 mM) histidine, about 60 mg/mL to about 200 mg/mL sucrose, and about 1.8 mg/mL to about 2.8 mg/mL poloxamer 188;
g) about 70 mg/mL to about 130 mg/mL of protein (e.g., an anti-FXI/FXIa antibody), about 30 mM to about 40 mM (e.g., about 35 mM) histidine, about 80 mg/mL sucrose, and about 2.0 mg/mL poloxamer 188.

In some embodiments, the pharmaceutical composition comprises any one of the following:
(a) about 70 mg/mL to about 130 mg/mL anti-FXI/FXIa antibody or antigen-binding fragment thereof,
(b) about 35 mM histidine,
(c) about 80 mg/mL sucrose, and
(d) about 2 mg/mL poloxamer 188,

the pharmaceutical composition having a pH of about 7.0; or
   (a) about 70 mg/mL to about 130 mg/mL anti-FXI/FXIa antibody or antigen-binding fragment thereof,
   (b) about 35 mM histidine,
   (c) about 80 mg/mL sucrose, and
   (d) about 2 mg/mL poloxamer 188,
the pharmaceutical composition having a pH of about 6.7; or
   (a) about 70 mg/mL to about 130 mg/mL anti-FXI/FXIa antibody or antigen-binding fragment thereof,
   (b) about 35 mM histidine,
   (c) about 80 mg/mL sucrose, and
   (d) about 2 mg/mL poloxamer 188,
the pharmaceutical composition having a pH of about 7.3; or
   (a) about 70 mg/mL to about 130 mg/mL anti-FXI/FXIa antibody or antigen-binding fragment thereof,
   (b) about 30 mM histidine,
   (c) about 80 mg/mL sucrose, and
   (d) about 1.8 mg/mL poloxamer 188,
the pharmaceutical composition having a pH of about 7.0; or
   (a) about 70 mg/mL to about 130 mg/mL anti-FXI/FXIa antibody or antigen-binding fragment thereof,
   (b) about 30 mM histidine,
   (c) about 80 mg/mL sucrose, and
   (d) about 2.8 mg/mL poloxamer 188,
the pharmaceutical composition having a pH of about 7.0; or
   (a) about 70 mg/mL to about 130 mg/mL anti-FXI/FXIa antibody or antigen-binding fragment thereof,
   (b) about 40 mM histidine,
   (c) about 80 mg/mL sucrose, and
   (d) about 1.8 mg/mL poloxamer 188,
the pharmaceutical composition having a pH of about 7.0; or
   (a) about 70 mg/mL to about 130 mg/mL anti-FXI/FXIa antibody or antigen-binding fragment thereof,
   (b) about 40 mM histidine,
   (c) about 80 mg/mL sucrose, and
   (d) about 2.3 mg/mL poloxamer 188,
the pharmaceutical composition having a pH of about 7.0; or
   (a) about 70 mg/mL to about 130 mg/mL anti-FXI/FXIa antibody or antigen-binding fragment thereof,
   (b) about 40 mM histidine,
   (c) about 80 mg/mL sucrose, and
   (d) about 2.8 mg/mL poloxamer 188,
the pharmaceutical composition having a pH of about 7.0; or
   (a) about 70 mg/mL to about 130 mg/mL anti-FXI/FXIa antibody or antigen-binding fragment thereof,
   (b) about 35 mM histidine,
   (c) about 80 mg/mL sucrose, and
   (d) about 2.3 mg/mL poloxamer 188,
the pharmaceutical composition having a pH of about 7.0; or
   (a) about 70 mg/mL to about 130 mg/mL anti-FXI/FXIa antibody or antigen-binding fragment thereof,
   (b) about 35 mM histidine,
   (c) about 80 mg/mL sucrose, and
   (d) about 1.8 mg/mL poloxamer 188,
the pharmaceutical composition having a pH of about 7.0.

### Other buffer systems

The present disclosure provides a pharmaceutical composition, which comprises an anti-FXI/FXIa antibody and a buffer. In some embodiments, the buffer is selected from the group consisting of an acetate buffer, a histidine salt buffer, a Tris-hydrochloride buffer, a Tris-citrate buffer, and a phosphate buffer. The composition has therapeutic or prophylactical activity. In addition, the composition may also have the advantages of good stability and the like.

In some embodiments, the pharmaceutical composition has a pH of about 4.0 to about 8.5, e.g., about 5.0 to about 8.0, about 6.0 to about 7.5, about 6.5 to about 7.5, about 6.6 to about 7.4, about 6.7 to about 7.3, about 6.8 to about 7.2, about 6.9 to about 7.1 (e.g., about 7.0), or any range between these point values. Some non-limiting examples of pH include about 4.0, about 4.5, about 5.0, about 6.0, about 6.4, about 6.5, about 6.6, about 6.7, about 6.8, about 6.9, about 7.0, about 7.1, about 7.2, about 7.3, about 7.4, about 7.5, about 7.6, about 7.7, about 7.8, about 7.9, about 8.0, about 8.1, about 8.2, about 8.3, about 8.4, and about 8.5.

Generally, the pH of the pharmaceutical composition obtained by replacing the buffer is almost consistent with the pH of the buffer. Also, it is well known to those skilled in the art that in the process of pharmaceutical formulation, there may sometimes be a pH drift, but the pH drift of the pharmaceutical formulation is generally small (within a range of ±0.3). In some embodiments, the pH drift of the pharmaceutical formulation is within a range of ±0.1.

In some embodiments, provided is the pharmaceutical composition according to any one of the above, wherein the anti-FXI/FXIa antibody or the antigen-binding fragment thereof has a concentration of about 0.1 mg/mL to about 1000 mg/mL, e.g., about 1 mg/mL to about 500 mg/mL, about 1 mg/mL to about 300 mg/mL, about 10 mg/mL to about 400 mg/mL, about 20 mg/mL to about 350 mg/mL, about 30 mg/mL to about 300 mg/mL, about 40 mg/mL to about 250 mg/mL, about 50 mg/mL to about 200 mg/mL, about 60 mg/mL to about 180 mg/mL, about 50 mg/mL to about 150 mg/mL, about 60 mg/mL to about 150 mg/mL, about 70 mg/mL to about 130 mg/mL, about 80 mg/mL to about 120 mg/mL, about 90 mg/mL to about 110 mg/mL, about 1 mg/mL to about 300 mg/mL, about 1 mg/mL to about 200 mg/mL, or any range between these point values. In some embodiments, the anti-FXI/FXIa antibody has a concentration of 1 mg/mL to 100 mg/mL. In some embodiments, the anti-FXI/FXIa antibody has a concentration of 40 mg/mL to 100 mg/mL. In some non-limiting embodiments, the anti-FXI/FXIa antibody has a concentration of about 1 mg/mL, about 10 mg/mL, about 20 mg/mL, about 30 mg/mL, about 40 mg/mL, about 45 mg/mL, about 50 mg/mL, about 55 mg/mL, about 60 mg/mL, about 65 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, or about 200 mg/mL.

In some embodiments, provided is the pharmaceutical composition according to any one of the above, wherein the pharmaceutical composition comprises a surfactant. In some embodiments, the surfactant is a nonionic surfactant. In some embodiments, the surfactant is selected from the group consisting of poloxamer (e.g., poloxamer 188), polysorbate (e.g., polysorbate 20 or polysorbate 80), poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocaramide propyl-betaine, linoleinamide propyl-betaine, myristylamide propyl-betaine, palmitamide propyl-betaine, isostearamide propyl-betaine, myristylamide propyl-dimethylamine, palmitamide propyl-dimethylamine, isostearamide propyl-dimethylamine, sodium methyl cocoyl, sodium methyl oleyl taurate, polyethylene glycol, polypropylene glycol, copolymers of ethylene and propylene glycol, and the like. In some embodiments, the surfactant is polysorbate or poloxamer. In some embodiments, the surfactant is polysorbate 80, polysorbate 20, or poloxamer 188. In some embodiments, the surfactant is poloxamer 188.

In some embodiments, provided is the pharmaceutical composition according to any one of the above, wherein the surfactant has a concentration of 0.05 mg/mL to 10 mg/mL, e.g., 0.1 mg/mL to 10 mg/mL, 0.05 mg/mL to 1.0 mg/mL, 0.1 mg/mL to 1.0 mg/mL, or 0.2 mg/mL to 0.8 mg/mL. In some embodiments, the surfactant has a concentration of 0.2 mg/mL to 0.6 mg/mL. In some embodiments, the surfactant has a concentration of 0.05 mg/mL, 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, or 1.0 mg/mL, or any range between these point values. In some embodiments, the surfactant has a concentration of 0.4 mg/mL. In some embodiments, the surfactant is 0.4mg/mL poloxamer 188. In some embodiments, the surfactant is 0.4 mg/mL polysorbate 80.

In some embodiments, provided is the pharmaceutical composition according to any one of the above, which comprises an osmotic pressure regulator. In some embodiments, the osmotic pressure regulator is a sugar (including monosaccharide, disaccharide, trisaccharide, polysaccharide, sugar alcohol, reducing sugar, non-reducing sugar, and the like), an amino acid (including arginine, glycine, cysteine, histidine, and the like), or a salt (sodium chloride, potassium chloride, calcium chloride, and the like). In some embodiments, the osmotic pressure regulator is a sugar, and the sugar is selected from the group consisting of glucose, sucrose, trehalose, lactose, fructose, maltose, dextran, glycerin, erythritol, glycerol, arabitol, sylitol, sorbitol (also known as sorbol), mannitol, melibiose, melezitose, raffinose, mannotriose, stachyose, maltose, lactulose, maltulose, maltitol, lactitol, and iso-maltulose. In some embodiments, the osmotic pressure regulator is selected from the group consisting of one or more of the group consisting of sucrose, trehalose, sorbitol, arginine, proline, glycine, and sodium chloride. In some embodiments, the osmotic pressure regulator is a non-reducing disaccharide. In some embodiments, the osmotic pressure regulator is trehalose or sucrose. In some embodiments, the osmotic pressure regulator is sucrose. In some embodiments, the osmotic pressure regulator is selected from the group consisting of sucrose, proline, and sucrose, or glycine and sucrose.

In some embodiments, provided is the pharmaceutical composition according to any one of the above, wherein the osmotic pressure regulator has a concentration of 1 mg/mL to 300 mg/mL, e.g., about 5 mg/mL to about 200 mg/mL, about 10 mg/mL to about 150 mg/mL, about 20 mg/mL to about 140 mg/mL, about 30 mg/mL to about 130 mg/mL, about 40 mg/mL to about 120 mg/mL, about 50 mg/mL to about 110 mg/mL, about 60 mg/mL to about 100 mg/mL, about 75 mg/mL to about 100 mg/mL, about 70 mg/mL to about 90 mg/mL, about 75 mg/mL to about 85 mg/mL, about 75 mg/mL to about 80 mg/mL, about 1 mg/mL to about 100 mg/mL, about 1 mg/mL to about 80 mg/mL, about 1 mg/mL to about 40 mg/mL, or any range between these point values. In some embodiments, non-limiting examples of the concentration of the osmotic pressure regulator include about 7.5 mg/mL, about 11.5 mg/mL, about 40 mg/mL, about 60 mg/mL, about 65 mg/mL, about 70 mg/mL, about 75 mg/mL, about 80 mg/mL, about 85 mg/mL, about 90 mg/mL, about 95 mg/mL, or about 100 mg/mL. In some embodiments, the pharmaceutical composition described above is an isotonic formulation. In some embodiments, the osmotic pressure regulator is 80 mg/mL sucrose. In some embodiments, the osmotic pressure regulator is 40 mg/mL sucrose. In some embodiments, the osmotic pressure regulator is about 10 mM to about 300 mM proline and about 1 mg/mL to about 200 mg/mL sucrose. In some embodiments, the osmotic pressure regulator is about 10 mM to about 200 mM proline and about 10 mg/mL to about 100 mg/mL sucrose. In some embodiments, the osmotic pressure regulator is about 50 mM to about 150 mM proline and about 10 mg/mL to about 80 mg/mL sucrose. In some embodiments, the osmotic pressure regulator is about 80 mM to about 120 mM proline and about 40 mg/mL to about 80 mg/mL sucrose. In some embodiments, the osmotic pressure regulator is about 100 mM proline and about 40 mg/mL sucrose. In some embodiments, the osmotic pressure regulator is about 10 mM to about 300 mM glycine and about 1 mg/mL to about 200 mg/mL sucrose. In some embodiments, the osmotic pressure regulator is about 10 mM to about 200 mM glycine and about 10 mg/mL to about 100 mg/mL sucrose. In some embodiments, the osmotic pressure regulator is about 50 mM to about 150 mM glycine and about 10 mg/mL to about 80 mg/mL sucrose. In some embodiments, the osmotic pressure regulator is about 80 mM to about 120 mM glycine and about 40 mg/mL to about 80 mg/mL sucrose. In some embodiments, the osmotic pressure regulator is about 100 mM glycine and about 40 mg/mL sucrose.

In some embodiments, provided is the pharmaceutical composition according to any one of the above, wherein the buffer has a concentration of about 5 mM to about 100 mM. In some embodiments, the buffer has a concentration of about 10 mM to about 30 mM. In some embodiments, the buffer has a concentration of about 5 mM to about 15 mM. In some embodiments, the buffer has a concentration of about 5 mM, about 10 mM, about 15 mM, about 20 mM, about 25 mM, about 30 mM, about 40 mM, about 50 mM, about 60 mM, about 70 mM, about 80 mM, about 90 mM, or about 100 mM, and any range between these point values. In some embodiments, the buffer is about 10 mM histidine-hydrochloride. In some embodiments, the buffer is about 10 mM sodium dihydrogen phosphate-disodium hydrogen phosphate. In some embodiments, the buffer is about 10 mM Tris-hydrochloric acid.

In some embodiments, the pharmaceutical composition according to any one of the above comprises any one of a)-i):
a) about 1 mg/mL to about 300 mg/mL protein (e.g., an anti-FXI/FXIa antibody) and about 5 mM to about 100 mM, about 10 mM to about 30 mM, or about 5 mM to about 15 mM (e.g., about 10 mM) histidine-hydrochloride, sodium dihydrogen phosphate-disodium hydrogen phosphate, or Tris-hydrochloric acid;
b) about 50 mg/mL to about 150 mg/mL protein (e.g., an anti-FXI/FXIa antibody) and about 5 mM to about 100 mM, about 10 mM to about 30 mM, or about 5 mM to about 15 mM (e.g., about 10 mM) histidine-hydrochloride, sodium dihydrogen phosphate-disodium hydrogen phosphate, or Tris-hydrochloric acid;
c) about 70 mg/mL to about 130 mg/mL protein (e.g., an anti-FXI/FXIa antibody) and about 5 mM to about 100 mM, about 10 mM to about 30 mM, or about 5 mM to about 15 mM (e.g., about 10 mM) histidine-hydrochloride, sodium dihydrogen phosphate-disodium hydrogen phosphate, or Tris-hydrochloric acid;
   wherein, the pharmaceutical composition in a)-c) has a pH of about 4.0 to about 8.5 (e.g., about 6.5 to about 7.5, e.g., about 7.0);
   optionally, the pharmaceutical composition in a)-c) further comprises sucrose, or trehalose, or glycine and sucrose, or proline and sucrose, and poloxamer 188 or polysorbate (e.g., polysorbate 80 or polysorbate 20);
   in some embodiments, the pharmaceutical composition in a)-c) further comprises sucrose or trehalose having a concentration of, e.g., about 1 mg/mL to about 300 mg/mL, about 10 mg/mL to about 150 mg/mL, or about 75 mg/mL to about 100 mg/mL (e.g., about 70 mg/mL, about 80 mg/mL, about 90 mg/mL, about 100 mg/mL, about 110 mg/mL, or about 120 mg/mL);
   in some embodiments, the pharmaceutical composition in a)-c) further comprises glycine and sucrose, e.g., about 10 mM to about 300 mM glycine and about 1 mg/mL to about 200 mg/mL sucrose, about 10 mM to about 200 mM glycine and about 10 mg/mL to about 100 mg/mL sucrose, or about 80 mM to about 120 mM glycine and about 40 mg/mL to about 80 mg/mL sucrose (e.g., about 100 mM glycine and about 40 mg/mL sucrose, about 90 mM glycine and about 50 mg/mL sucrose, or about 110 mM glycine and about 30 mg/mL sucrose);
   in some embodiments, the pharmaceutical composition in a)-c) further comprises proline and sucrose, e.g., about 10 mM to about 300 mM proline and about 1 mg/mL to about 200 mg/mL sucrose, about 10 mM to about 200 mM proline and about 10 mg/mL to about 100 mg/mL sucrose, or about 80 mM to about 120 mM proline and about 40 mg/mL to about 80 mg/mL sucrose (e.g., about 100 mM proline and about 40 mg/mL sucrose, about 90 mM proline and about 50 mg/mL sucrose, or about 110 mM proline and about 30 mg/mL sucrose);
   in some embodiments, the pharmaceutical compositions in a)-c) further comprises poloxamer 188 or polysorbate (e.g., polysorbate 80 or polysorbate 20) having a concentration of about 0.1 mg/mL to about 10 mg/mL, about 1.5 mg/mL to about 4.0 mg/mL, about 1.8 mg/mL to about 3.5 mg/mL, or about 1.8 mg/mL to about 2.8 mg/mL (e.g., about 1.8 mg/mL, about 1.9 mg/mL, about 2.0 mg/mL, about 2.1 mg/mL, about 2.2 mg/mL, about 2.3 mg/mL, about 2.4 mg/mL, about 2.5 mg/mL, about 2.6 mg/mL, about 2.7 mg/mL, or about 2.8 mg/mL);
d) about 1 mg/mL to about 300 mg/mL protein (e.g., an anti-FXI/FXIa antibody or an antigen-binding fragment thereof) and about 10 mM histidine-hydrochloride, sodium dihydrogen phosphate-disodium hydrogen phosphate, or Tris-hydrochloric acid, the pharmaceutical composition having a pH of about 6.5 to about 7.5; optionally, the pharmaceutical composition may further comprise about 80 mg/mL sucrose and about 0.1 mg/mL to about 10 mg/mL poloxamer 188 or polysorbate;
e) about 1 mg/mL to about 300 mg/mL protein (e.g., an anti-FXI/FXIa antibody or an antigen-binding fragment thereof) and about 10 mM histidine-hydrochloride, sodium dihydrogen phosphate-disodium hydrogen phosphate, or Tris-hydrochloric acid, the pharmaceutical composition having a pH of about 6.5 to about 7.5; optionally, the pharmaceutical composition may further comprise about 100 mM glycine or proline, about 40 mg/mL sucrose, and about 0.1 mg/mL to about 10 mg/mL poloxamer 188 or polysorbate;
f) about 50 mg/mL to about 150 mg/mL protein (e.g., an anti-FXI/FXIa antibody or an antigen-binding fragment thereof) and about 5 mM to about 100 mM histidine-hydrochloride, sodium dihydrogen phosphate-disodium hydrogen phosphate, or Tris-hydrochloric acid, the pharmaceutical composition having a pH of about 4.0 to about 8.5; optionally, the pharmaceutical composition may further comprise about 10 mg/mL to about 150 mg/mL sucrose and about 0.1 mg/mL to about 10 mg/mL poloxamer 188 or polysorbate;
g) about 50 mg/mL to about 150 mg/mL protein (e.g., an anti-FXI/FXIa antibody or an antigen-binding fragment thereof) and about 5 mM to about 100 mM histidine-hydrochloride, sodium dihydrogen phosphate-disodium hydrogen phosphate, or Tris-hydrochloric acid, the pharmaceutical composition having a pH of about 4.0 to about 8.5; optionally, the pharmaceutical composition may further comprise about 10 mM to about 200 mM glycine or proline, about 10 mg/mL to about 100 mg/mL sucrose, and about 0.1 mg/mL to about 10 mg/mL poloxamer 188 or polysorbate;
h) about 70 mg/mL to about 130 mg/mL protein (e.g., an anti-FXI/FXIa antibody or an antigen-binding fragment thereof) and about 5 mM to about 15 mM histidine-hydrochloride, sodium dihydrogen phosphate-disodium hydrogen phosphate, or Tris-hydrochloric acid, the pharmaceutical composition having a pH of about 6.5 to about 7.5; optionally, the pharmaceutical composition may further comprise about 80 mg/mL sucrose and about 1.5 mg/mL to about 4 mg/mL poloxamer 188 or polysorbate;
i) about 70 mg/mL to about 130 mg/mL protein (e.g., an anti-FXI/FXIa antibody or an antigen-binding fragment thereof) and about 5 mM to about 15 mM histidine-hydrochloride, sodium dihydrogen phosphate-disodium hydrogen phosphate, or Tris-hydrochloric acid, the pharmaceutical composition having a pH of about 6.5 to about 7.5; optionally, the pharmaceutical composition may further comprise about 100 mM glycine or proline, about 40 mg/mL sucrose, and about 1.5 mg/mL to about 4 mg/mL poloxamer 188 or polysorbate.

The pharmaceutical composition of the present disclosure further comprises a solvent. The solvent in the pharmaceutical composition is selected from, without limitation, non-toxic physiologically acceptable liquid carriers, such as normal saline, water for injection, or a glucose solution (e.g. 5% glucose injection or glucose-sodium chloride injection).

The present disclosure further provides a pharmaceutical composition, which is obtained by diluting the pharmaceutical composition described above with 0.9% normal saline or 5% glucose solution or which comprises an anti-FXI/FXIa antibody or an antigen-binding fragment thereof, histidine, sucrose, and poloxamer 188 each at a concentration suitable for intravenous injection obtained after diluting the pharmaceutical composition described above with 0.9% normal saline or 5% glucose solution. In some embodiments, the anti-FXI/FXIa antibody or the antigen-binding fragment thereof in the pharmaceutical composition has a concentration of about 0.01 mg/mL to about 50 mg/mL. In some embodiments, the anti-FXI/FXIa antibody or the antigen-binding fragment thereof in the pharmaceutical composition has a concentration of about 0.1 mg/mL to about 30 mg/mL. In some embodiments, the anti-FXI/FXIa antibody or the antigen-binding fragment thereof in the pharmaceutical composition has a concentration of about 0.20 mg/mL to about 13.30 mg/mL.

In some embodiments, the protein in the pharmaceutical composition of the present disclosure is an antibody, an antibody fragment, or an Fc fusion protein. In some embodiments, the antigen targeted by the antibody, the antibody fragment, or the Fc fusion protein is selected from the group consisting of: factor VIIIC, factor IX, factor XI/factor XIa (FXI/FXIa), tissue factor, vascular endothelial growth factor (VEGF), renin, growth hormone, growth hormone releasing factor, parathyroid hormone, thyroid stimulating hormone, lipoprotein, α-1-antitrypsin, bone-derived neurotrophic factor (BDNF), neurotrophin-3, -4, -5, or -6 (NT-3, NT-4, NT-5, or NT-6), epidermal growth factor (EGF), transforming growth factor (TGF), interferon, M-CSF, GM-CSF, G-CSF, IL-1 to IL-10, HER2, HER3 or HER4 receptor, CTLA-4, PD-1, PD-L1, PD-L2, TIM3, 41BB, 41BBL, OX40, OX40L, GITRL, GITR, CD25, CD27, CD70, CD80, TNFRSF25, CD40L, CD40, B7 H3, B7 H4, VISTA, TMIGD2, HHLA2-TMIGD2, butyrophilin (including BTNL2), Siglec family, TIGIT and PVR family members, KIRs, ILTs and LIRs, CD79, GARP, GITR, PSMA, neuropilin, CD160, CD30, and CD155, and fragments of any of the above polypeptides. In some embodiments, the protein in the pharmaceutical composition is an anti-FXI/FXIa antibody or an antigen-binding fragment thereof.

In some embodiments, the anti-FXI/FXIa antibody or the antigen-binding fragment thereof in the pharmaceutical composition described above comprises a heavy chain variable region and a light chain variable region, wherein:
the heavy chain variable region comprises a HCDR1, a HCDR2, and a HCDR3 set forth in SEQ ID NOs: 7, 8, and 9, respectively, and the light chain variable region comprises a LCDR1, a LCDR2, and a LCDR3 set forth in SEQ ID NOs: 10, 11, and 12.

In some embodiments, the anti-FXI/FXIa antibody or the antigen-binding fragment thereof in the pharmaceutical composition may be selected from the group consisting of a murine antibody, a chimeric antibody, and a humanized antibody, e.g., a humanized antibody.

In an optional embodiment, the light and heavy chain FR sequences in the light and heavy chain variable regions of the humanized anti-FXI/FXIa antibody in the pharmaceutical composition are derived from human germline light and heavy chain FRs or mutated sequences thereof, respectively. In some embodiments, the anti-FXI/FXIa antibody or the antigen-binding fragment thereof in the pharmaceutical composition comprises an antibody heavy chain variable region and an antibody light chain variable region, wherein:
the sequence of the heavy chain variable region is set forth in SEQ ID NO: 5 or a sequence having at least 80%, at least 85%, or at least 90% identity thereto, and the sequence of the light chain variable region is set forth in SEQ ID NO: 6 or a sequence having at least 80%, at least 85%, or at least 90% identity thereto;
the sequence of the heavy chain variable region is set forth in SEQ ID NO: 13 or a sequence having at least 80%, at least 85%, or at least 90% identity thereto, and the sequence of the light chain variable region is set forth in SEQ ID NO: 14 or a sequence having at least 80%, at least 85%, or at least 90% identity thereto;
the sequence of the heavy chain variable region is set forth in SEQ ID NO: 15 or a sequence having at least 80%, at least 85%, or at least 90% identity thereto, and the sequence of the light chain variable region is set forth in SEQ ID NO: 16 or a sequence having at least 80%, at least 85%, or at least 90% identity thereto;
the sequence of the heavy chain variable region is set forth in SEQ ID NO: 17 or a sequence having at least 80%, at least 85%, or at least 90% identity thereto, and the sequence of the light chain variable region is set forth in SEQ ID NO: 16 or a sequence having at least 80%, at least 85%, or at least 90% identity thereto.

In some specific embodiments, the anti-FXI/FXIa antibody or the antigen-binding fragment thereof in the pharmaceutical composition comprises an antibody heavy chain variable region and an antibody light chain variable region, wherein the sequence of the heavy chain variable region is set forth in SEQ ID NO: 17, and the sequence of the light chain variable region is set forth in SEQ ID NO: 16.

In some embodiments, the anti-FXI/FXIa antibody or the antigen-binding fragment thereof further comprises a heavy chain constant region and a light chain constant region. In an optional embodiment, the heavy chain constant region is selected from the group consisting of human IgG1, IgG2, IgG3, IgG4, and IgG4P (i.e., the S241P mutant of IgG4) constant regions, and the light chain constant region is selected from the group consisting of human kappa and lambda chain constant regions. The sequence of the IgG4P Fc (i.e., IgG4 Fc comprising S241P) is set forth in SEQ ID NO: 19. In some embodiments, the sequence of the heavy chain CH1 is set forth in SEQ ID NO: 18 or a sequence having at least 80%, at least 85%, or at least 90% identity thereto, and/or the sequence of the light chain constant region is set forth in SEQ ID NO: 20 or a sequence having at least 80%, at least 85%, or at least 90% identity thereto.

In some embodiments, the anti-FXI/FXIa antibody or the antigen-binding fragment thereof comprises a heavy chain and a light chain, wherein the heavy chain has the sequence set forth in SEQ ID NO: 21 or a sequence having at least 80%, at least 85%, or at least 90% identity thereto, and the light chain has the sequence set forth in SEQ ID NO: 22 or a sequence having at least 80%, at least 85%, or at least 90% identity thereto.

In the present disclosure, the "at least 90% identity" encompasses at least 90%, at least 95%, at least 96%, at least 97%, at least 98%, and at least 99% identity.

In the present disclosure, the "at least 90% identity" encompasses at least 80%, at least 85%, at least 87%, at least 88%, at least 89%, at least 90%, at least 95%, at least 96%, at least 97%, at least 98%, and at least 99% identity.

In some specific embodiments, the anti-FXI/FXIa antibody in the pharmaceutical composition comprises an antibody heavy chain and an antibody light chain, wherein the sequence of the heavy chain is set forth in SEQ ID NO: 21, and the sequence of the light chain is set forth in SEQ ID NO: 22.

In some embodiments, the antigen-binding fragment of the anti-FXI/FXIa antibody is a Fab, an Fv, an sFv, a Fab', an F(ab')2, a linear antibody, a single-chain antibody, an scFv, an sdAb, an sdFv, a nanobody, a peptibody, a domain antibody, and a multispecific antibody (a bispecific antibody, a diabody, a triabody, a tetrabody, a tandem di-scFv, and a tandem tri-scFv), e.g., specifically, an scFv, an Fv, a Fab, or a Fab' fragment.

In some embodiments, the heavy chain variable region and/or the light chain variable region of the anti-FXI/FXIa antibody comprises 1, 2, 3, 4, 5, 6, 7, 8, 9, or 10 amino acid changes. The amino acid change may be a conservative replacement of an amino acid residue in the variable region.

In some embodiments, the anti-FXI/FXIa antibody described above may have any one or more of the following properties:
preventing activation of the intrinsic or common coagulation pathway;
blocking binding of FXI and/or FXIa to one or more of blood coagulation factor IX, blood coagulation factor XIIa, or thrombin and other components of the coagulation pathway;
blocking binding of one or more of FIX, FXI, and FXIa to a platelet receptor;
having a KD for binding to a human FXI and/or FXIa protein of ≤ 10⁻⁹;
preventing the FXI/FXIa catalytic domain from presenting in an active conformation when binding to FXI/FXIa; and
having the ability to be used for subcutaneous administration or intravenous administration.

The affinity described above is measured, for example, by BIACORE^{™}.

In some embodiments, the pharmaceutical composition according to any one of the above may be an intravenous injection, a subcutaneous injection, an intraperitoneal injection, or an intramuscular injection.

In some embodiments, the present disclosure provides a method for preparing the pharmaceutical composition according to any one of the above, which comprises the step of subjecting a stock solution of the anti-FXI/FXIa antibody or the antigen-binding fragment thereof to buffer-exchanging. The present disclosure further provides a liquid formulation, which comprises the pharmaceutical composition according to any one of the above.

The pharmaceutical composition described in the present disclosure already has sufficient stability for being prepared into a drug and can be stored stable for a long time.

The present disclosure further provides a method for preparing a lyophilized formulation of the pharmaceutical composition comprising the anti-FXI/FXIa antibody, which comprises the step of lyophilizing the pharmaceutical composition described above.

The present disclosure further provides a lyophilized formulation of the pharmaceutical composition comprising the anti-FXI/FXIa antibody, which is obtained by lyophilizing the pharmaceutical composition according to any one of the above.

In some embodiments, the lyophilized formulation is stored in a dark place at 2-8 °C and is stable for at least 1 month, at least 3 months, at least 6 months, at least 12 months, at least 18 months, at least 24 months, or at least 30 months.

In some embodiments, the lyophilized formulation is stable at 25 °C for at least 1 month, at least 3 months, at least 6 months, or at least 12 months.

In some embodiments, the lyophilized formulation is stable at 40 °C for at least 7 days, at least 14 days, or at least 30 days.

The present disclosure further provides a reconstituted solution comprising the anti-FXI/FXIa antibody pharmaceutical composition, wherein the reconstituted solution is obtained by reconstituting the lyophilized formulation according to any one of the above.

The present disclosure further provides an article of manufacture, which comprises a container containing the pharmaceutical composition, the liquid formulation, the lyophilized formulation, or the reconstituted solution according to any one of the above. In some embodiments, the container may be, but is not limited to, a tubular injection vial made of neutral borosilicate glass.

The present disclosure further provides use of the pharmaceutical composition according to any one of the above, the liquid formulation according to any one of the above, the lyophilized formulation according to any one of the above, the reconstituted solution according to any one of the above, or the article of manufacture according to any one of the above in the preparation of a medicament for treating a disease.

The present disclosure further provides a method for treating or preventing a disease, which comprises administering to a subject in need thereof an effective amount of the pharmaceutical composition according to any one of the above, the liquid formulation according to any one of the above, the lyophilized formulation according to any one of the above, the reconstituted solution according to any one of the above, or the article of manufacture according to any one of the above.

The present disclosure further provides the pharmaceutical composition according to any one of the above, the liquid formulation according to any one of the above, the lyophilized formulation according to any one of the above, the reconstituted solution according to any one of the above, or the article of manufacture according to any one of the above for use in treating a disease.

In some embodiments, the disease according to any one of the above is a thrombotic or thromboembolic disease and/or a thrombotic or thromboembolic complication, cardiac arrhythmia, cardiogenic thromboembolism, or disseminated intravascular coagulation.

In some embodiments, with respect to the disease according to any one of the above, the thrombotic or thromboembolic disease is selected from, without limitation, cardiac coronary artery diseases, such as acute coronary syndrome (ACS), ST-elevation myocardial infarction (STEMI), non-ST-elevation myocardial infarction (non-STEMI), stable angina pectoris, unstable angina pectoris, and reocclusion and restenosis after coronary interventions, thrombotic or thromboembolic diseases leading to peripheral arterial occlusive disease, pulmonary embolism, venous thromboembolism, venous thrombosis, transient ischemic attack, thrombotic stroke, and thromboembolic stroke in other blood vessels, pulmonary disease and pulmonary hypertension caused by chronic thromboembolism (CTEPH), and a combination thereof.

The present disclosure provides a method for treating or preventing a disease, which comprises administering to a subject in need thereof a therapeutically or prophylactically effective amount of the pharmaceutical composition according to any one of the above, the liquid formulation according to any one of the above, the lyophilized formulation according to any one of the above, the reconstituted solution according to any one of the above, or the article of manufacture according to any one of the above, wherein the disease is a thrombotic or thromboembolic disease and/or a thrombotic or thromboembolic complication, cardiac arrhythmia, cardiogenic thromboembolism, or disseminated intravascular coagulation. In some embodiments, the thrombotic or thromboembolic disease or the complication thereof is selected from, without limitation, cardiac coronary artery diseases, such as acute coronary syndrome (ACS), ST-elevation myocardial infarction (STEMI), non-ST-elevation myocardial infarction (non-STEMI), stable angina pectoris, unstable angina pectoris, and reocclusion and restenosis after coronary interventions, thrombotic or thromboembolic diseases leading to peripheral arterial occlusive disease, pulmonary embolism, venous thromboembolism, venous thrombosis, transient ischemic attack, thrombotic stroke, and thromboembolic stroke in other blood vessels, pulmonary disease and pulmonary hypertension caused by chronic thromboembolism (CTEPH), and a combination thereof. In some embodiments, the administration is performed by subcutaneous or intravenous injection.

### BRIEF DESCRIPTION OF THE DRAWINGS

FIG. 1 shows the experiment on inhibition of FXIIa-mediated FXI activating enzyme activity *in vitro* by anti-FXI/FXIa antibody.
FIG. 2 shows the determination of aPTT and PT anticoagulant activity in human blood, wherein FIG. 2A and FIG. 2B show the experimental results of aPTT and PT of the anti-FXI/FXIa antibodies in the human blood, respectively. 1209 was another anti-FXI/FXIa antibody obtained by screening in the present application.
FIG. 3 shows the determination of aPTT and PT anticoagulant activity in monkey blood, wherein FIG. 3A and FIG. 3B show the experimental results of aPTT and PT of the anti-FXI/FXIa antibodies in the monkey blood, respectively.
FIG. 4 shows the animal experiment of anti-FXI/FXIa antibodies in cynomolgus monkeys, wherein FIG. 4A shows curves of change in aPTT, plasma drug concentration, FXI:C%, and free FXI of antibody 3882 in cynomolgus monkeys, FIG. 4B shows the inhibition effect of 3882 on thrombosis in cynomolgus monkeys, and FIGs. 4C and 4D show safety tests of 3882 in cynomolgus monkeys, with bleeding time determination and PT detection results shown, respectively.
FIG. 5 shows the results of the pharmacodynamic (PD) experiment of anti-FXI/FXIa antibodies in cynomolgus monkeys, wherein FIG. 5A shows the detection results of APTT(s), and FIG. 5B shows detection results of FXI:C (%); 3882 (1 mg/kg) was administered intravenously and subcutaneously, and the control BAY1213790 (1 mg/kg) was administered intravenously.
FIG. 6 shows the screening for pH of buffer system in anti-FXI/FXIa antibody formulation, wherein FIGs. 6A, 6B, and 6C show the change trends of SEC, NRCE, and iCIEF, respectively, under conditions of different pH/buffers and 40 °C.
FIG. 7 shows the screening for pH, auxiliary material, and surfactant concentration in anti-FXI/FXIa antibody formulation, wherein FIGs. 7A, 7B, and 7C show the change trends of SEC, NRCE, and CEX, respectively, under conditions of different pH/buffers and 40 °C.
FIG. 8 shows the screening for buffer system and surfactant type of anti-FXI/FXIa antibody formulation, wherein FIGs. 8A and 8B show the change trends of SEC and CEX, respectively, under conditions of 40 °C.

### DETAILED DESCRIPTION

### Terminology

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains.

"Buffer" refers to a buffer that resists changes in pH by the action of its acid-base conjugate components. Examples of buffers that maintain the pH within an appropriate range include acetate, succinate, gluconate, histidine, oxalate, lactate, phosphate, citrate, tartrate, fumarate, glycylglycine, and other organic acid buffers.

"Histidine buffer" is a buffer comprising histidine ions. Examples of the histidine buffer include histidine-hydrochloride, histidine-acetate, histidine-phosphate, histidine-sulfate, and other buffers; histidine-hydrochloride buffer is preferred. The histidine-hydrochloride buffer is prepared with histidine and hydrochloric acid, or with histidine and histidine hydrochloride.

"Self-buffering" refers to the ability of a substance, such as a pharmaceutical protein (e.g., an antibody or an antigen-binding fragment thereof; in some embodiments, an anti-FXI/FXIa antibody, e.g., 3882 antibody of the present disclosure), to sufficiently resist pH changes resulting from a given application in the absence of other buffers.

"Tris-citrate buffer" is a buffer that comprises citrate ions. Examples of the Tris-citrate buffer include Tris-hydrochloride, Tris-acetate, Tris-phosphate, Tris-sulfate, Tris-citrate, and other buffers; Tris-citrate is preferred.

"Tris-hydrochloride buffer" is a buffer comprising hydrochloride ions. Examples of the Tris-hydrochloride buffer include Tris-hydrochloride, Tris-acetate, Tris-phosphate, Tris-sulfate, Tris-citrate, and other buffers. The preferred citrate buffer is Tris-hydrochloride buffer.

"Phosphate buffer" is a buffer comprising phosphate ions. Examples of the phosphate buffer include disodium hydrogen phosphate-sodium dihydrogen phosphate, disodium hydrogen phosphate-potassium dihydrogen phosphate, and the like. The preferred phosphate buffer is the disodium hydrogen phosphate-sodium dihydrogen phosphate buffer.

"Acetate buffer" is a buffer comprising acetate ions. Examples of the acetate buffer include acetic acid-sodium acetate, acetic acid-histidine salt, acetic acid-potassium acetate, acetic acid-calcium acetate, acetic acid-magnesium acetate, and the like. The preferred acetate buffer is the acetic acid-sodium acetate buffer.

"Pharmaceutical composition" refers to a mixture containing one or more of the compounds described herein or physiologically/pharmaceutically acceptable salts or prodrugs thereof, and other chemical components, wherein the other components are, for example, physiologically/pharmaceutically acceptable carriers and excipients. The pharmaceutical composition is intended to promote administration to an organism and facilitate the absorption of the active ingredient so that it can exert its biological activity. As used herein, "pharmaceutical composition" and "formulation" are not mutually exclusive.

Unless otherwise specified, the solvent in the pharmaceutical composition described in the present disclosure in solution form is water.

The term "about" as used herein means that a numerical value is within an acceptable error range for the particular value determined by one of ordinary skill in the art, and the numerical value depends in part on how the value is measured or determined (i.e., the limits of the measurement system). For example, "about" may mean a standard deviation within 1 or more than 1 in each practice in the art. Or, "about" or "substantially comprising" may mean a range of up to 20%. Furthermore, particularly for biological systems or processes, the term may mean up to an order of magnitude or up to 5-fold of a numerical value. Unless otherwise specified, when a specific value is provided in the present application and claims, the meaning of "about" or "substantially comprising" should be assumed to be within an acceptable error range for that specific value.

The pharmaceutical composition described in the present disclosure can achieve the effect of being stable: the antibody in the pharmaceutical composition substantially retains its physical and/or chemical stability and/or biological activity after storage; preferably, the pharmaceutical composition substantially retains its physical and chemical stability as well as its biological activity after storage.

The storage period is generally selected based on a predetermined shelf life of the pharmaceutical composition. There are a variety of analytical techniques currently available for measuring protein stability, and the stability after storage for a selected period of time at a selected temperature can be measured.

A stable pharmaceutical antibody formulation is one in which no significant change is observed under the following conditions: stored at refrigeration temperature (2-8 °C) for at least 3 months, preferably 6 months, more preferably 1 year, and even more preferably at most 2 years. In addition, stable liquid formulations include liquid formulations that exhibit desirable features after storage at 25 °C for periods including 1 month, 3 months, and 6 months, or storage at 40 °C for periods including 1 month. Typical acceptable criteria for stability are as follows: typically, no more than about 10%, preferably no more than about 5%, of antibody monomer is degraded as measured by SEC-HPLC. The pharmaceutical antibody formulation is colorless or yellow, or clear to slightly opalescent, by visual analysis. The concentration, pH, and osmolality of the formulation have a change of no more than ±10%. Typically, clippings of no more than about 10%, preferably no more than about 5%, are observed. Typically, aggregation of no more than about 10%, preferably no more than about 5%, is formed.

An antibody "retains its physical stability" in a pharmaceutical formulation if it shows no significant increase in aggregation, precipitation, and/or denaturation upon visual inspection of color and/or clarity, or as determined by UV light scattering, size exclusion chromatography (SEC), and dynamic light scattering (DLS). Changes in protein conformation can be evaluated by fluorescence spectroscopy (which determines the protein tertiary structure) and by FTIR spectroscopy (which determines the protein secondary structure).

An antibody "retains its chemical stability" in a pharmaceutical formulation if it shows no significant chemical change. Chemical stability can be evaluated by detecting and quantifying chemically changed protein. Degradation processes that often change the chemical structure of proteins include hydrolysis or clipping (assessed by methods such as size exclusion chromatography and SDS-PAGE), oxidation (assessed by methods such as peptide mapping in combination with mass spectroscopy or MALDI/TOF/MS), deamidation (assessed by methods such as ion-exchange chromatography, capillary isoelectric focusing, peptide mapping, and isoaspartic acid determination), and isomerization (assessed by isoaspartic acid content determination, peptide mapping, etc.).

An antibody "retains its biological activity" in a pharmaceutical formulation if the biological activity of the antibody at a given time is within a predetermined range of the biological activity exhibited during the preparation of the pharmaceutical formulation. The biological activity of an antibody can be determined, for example, by an antigen-binding assay.

"Factor XI", also referred to herein as "coagulation factor XI", "FXI", or "fXI", is a double-stranded glycoprotein having a molecular weight of about 160 kilodaltons (kD). Both chains may be identical polypeptides having a molecular weight of about 80,000 daltons and linked by disulfide bonds. FXI contains 4 "apple domains" (A1-A4 from the N-terminus, the heavy chain) and the C-terminal catalytic domain (the light chain). Without wishing to be bound by a particular theory, it is believed that the four apple domains contain FXI binding sites for other proteins, such as A1 for thrombin; A2 for HK; A3 for factor IX (FIX), GPIb, and heparin; and A4 for FXIIa. FXI can be converted to its active form, i.e., coagulation factor XIa (FXIa), by factor XIIa (FXIIa). The serine protease FXIa converts coagulation factor IX to IXa, which then activates coagulation factor X (Xa). Xa can then mediate the activation of coagulation factor II/thrombin.

The three-letter and single-letter codes for amino acids used in the present disclosure are as described in J. Biol. Chem, 243, p3558 (1968).

"Antibody" or "immunoglobulin" is used in the broadest sense and encompasses a variety of antibody structures, including but not limited to conventional antibodies (antibodies with a tetra-peptide-chain structure formed by linking two identical heavy chains and two identical light chains via inter-chain disulfide bonds) and a Fab, an Fv, an sFv, an F(ab')2, a linear antibody, a single-chain antibody, an scFv, an sdAb, an sdFv, a nanobody, a peptibody, a domain antibody (a heavy chain (VH) antibody and a light chain (VL) antibody) and a multispecific antibody (a bispecific antibody, a diabody, a triabody, a tetrabody, a tandem di-scFv, and a tandem tri-scFv) having antigen binding activity. The term "antibody" as used in the present disclosure includes full-length antibodies, individual chains thereof, any portions (i.e., antigen-binding fragments), domains, or fragments thereof having antigen binding activity, and multispecific antibodies comprising individual chains thereof and any portions, domains, or fragments thereof having antigen binding activity (including but not limited to antigen-binding domains or fragments, such as VHH domains or VH/VL domains). A conventional antibody or immunoglobulin is usually of a tetra-peptide-chain structure formed by linking two identical heavy chains and two identical light chains by inter-chain disulfide bonds. According to differences in the amino acid composition and the order of arrangement of the heavy chain constant regions, immunoglobulins can be divided into five classes, otherwise called isotypes of immunoglobulins, namely IgM, IgD, IgG, IgA, and IgE, with their corresponding heavy chains being µ chain, δ chain, γ chain, α chain, and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into κ or λ chains according to differences in the constant regions. Each of the five Ig classes may have a κ (kappa) chain or a λ (lambda) chain. In some embodiments, the antibody of the present disclosure specifically or substantially specifically binds to human FXI/FXIa.

In the heavy and light chains of the antibody, the sequences of about 110 amino acids near the N-terminus vary considerably and thus are referred to as variable regions (Fv regions); the remaining amino acid sequences near the C-terminus are relatively stable and thus are referred to as constant regions. The variable region comprises 3 hypervariable regions (HVRs) and 4 framework regions (FRs) with relatively conservative sequences. The 3 hypervariable regions determine the specificity of the antibody and thus are also known as complementarity determining regions (CDRs). Each light chain variable region (LCVR) or heavy chain variable region (HCVR) consists of 3 CDRs and 4 FRs arranged from the amino-terminus to the carboxyl-terminus in the following order: FR1, CDR1, FR2, CDR2, FR3, CDR3, and FR4. The 3 CDRs of the light chain refer to a LCDR1, a LCDR2, and a LCDR3, and the 3 CDRs of the heavy chain refer to a HCDR1, a HCDR2, and a HCDR3. The CDR amino acid residues of the LCVRs and HCVRs of the antibodies or antigen-binding fragments described in the present disclosure accord with the known Kabat numbering scheme (LCDR1-3, HCDR2-3), or accord with the Kabat and Chothia numbering schemes (HCDR1), in quantitative and positional terms.

The antibody of the present disclosure includes a murine antibody, a chimeric antibody, and a humanized antibody, and preferably a humanized antibody.

The term "chimeric antibody" refers to an antibody obtained by fusing a variable region of a murine antibody to a constant region of a human antibody, which can reduce an immune response induced by the murine antibody. The chimeric antibody is established by firstly establishing a hybridoma secreting a murine specific monoclonal antibody, then cloning a variable region gene from the mouse hybridoma cells, cloning a constant region gene of a human antibody as required, linking the mouse variable region gene and the human constant region gene to form a chimeric gene, inserting the chimeric gene into a human vector, and finally expressing a chimeric antibody molecule in a eukaryotic industrial system or prokaryotic industrial system. In a preferred embodiment of the present disclosure, the antibody light chain of the FXI chimeric antibody further comprises a light chain constant region of a human κ or λ chain or a variant thereof. The antibody heavy chain of the FXI chimeric antibody further comprises a heavy chain constant region of human IgG1, IgG2, IgG3 or IgG4 or a variant thereof.

The term "humanized antibody", also known as a CDR-grafted antibody, refers to an antibody produced by grafting mouse CDR sequences into a human antibody variable region framework, i.e., a different type of human germline antibody framework sequence. The undesired strong antibody response induced by the chimeric antibody as it carries a large amount of mouse protein components can be overcome. Such framework sequences can be obtained from public DNA databases or published references that include germline antibody gene sequences. For example, germline DNA sequences of genes of the human heavy and light chain variable regions can be found in the "VBase" human germline sequence database (available at the Internet address www.mrccpe.com.ac.uk/vbase), as well as in Kabat, E. A. et al., 1991 Sequences of Proteins of Immunological Interest, 5th edition. To avoid the decrease in activity caused by the decrease in immunogenicity, the FR sequence in the human antibody variable region can be subjected to minimum reverse mutation or back mutation to maintain activity. The humanized antibody of the present disclosure also includes humanized antibodies formed by further affinity maturation of the CDRs by phage display.

The "mutations" in the mutated sequences described in the present disclosure include, but are not limited to, "back mutation", "conservative modification", or "conservative replacement or substitution". The "conservative modification" or "conservative replacement or substitution" described in the present disclosure refers to replacement of amino acids in a protein with other amino acids having similar characteristics (e.g., charge, side-chain size, hydrophobicity/hydrophilicity, backbone conformation and rigidity, etc.), so that changes can be frequently made without changing the biological activity of the protein. Those skilled in the art know that, generally speaking, a single amino acid replacement in a non-essential region of a polypeptide does not substantially change the biological activity (see, e.g., Watson et al. (1987) Molecular Biology of the Gene, The Benjamin/Cummings Pub. Co., p224, (4th edition)). In addition, the replacement of amino acids with similar structure or function is unlikely to disrupt the biological activity.

The "mutated sequence" described in the present disclosure refers to a nucleotide sequence and/or amino acid sequence having a different degree of percent sequence identity to the nucleotide sequence and/or amino acid sequence of the present disclosure that is obtained when mutational modifications such as replacements, insertions, or deletions are appropriately made to the nucleotide sequence and/or amino acid sequence of the present disclosure. The sequence identity described in the present disclosure may be at least 85%, 90%, or 95%, for example, at least 95%. Non-limiting examples include 85%, 86%, 87%, 88%, 89%, 90%, 91%, 92%, 93%, 94%, 95%, 96%, 97%, 98%, 99%, and 100%. Sequence comparison and percent identity determination between two sequences can be performed by the default settings for the BLASTN/BLASTP algorithm available on the website National Center For Biotechnology Institute.

The term "binding to FXI/FXIa" herein refers to the ability to interact with FXI/FXIa or an epitope thereof, wherein the FXI/FXIa or the epitope thereof may be derived from human. The term "antigen-binding site" herein refers to a discontinuous three-dimensional spatial site on an antigen that is recognized by the antibody or the antigen-binding fragment of the present disclosure.

The term "specific binding" is determined by techniques available in the art, e.g., competitive ELISA, BIACORE^{®} assay, or KINEXA^{®} assay. The term is also applicable where, e.g., an antigen-binding domain of an antibody of the present disclosure is specific for a particular epitope that is carried by a number of antigens, in which case the antibody carrying the antigen-binding domain will be able to specifically bind to the various antigens carrying the epitope.

The "conservative modification" or "conservative replacement or substitution" refers to replacement of amino acids in a protein with other amino acids having similar characteristics (e.g., charge, side-chain size, hydrophobicity/hydrophilicity, backbone conformation and rigidity, etc.), so that changes can be frequently made without changing the biological activity of the protein. Those skilled in the art know that, generally speaking, a single amino acid replacement in a non-essential region of a polypeptide does not substantially change the biological activity (see, e.g., Watson et al. (1987) Molecular Biology of the Gene, The Benjamin/Cummings Pub. Co., p224, (4th edition)). In addition, the replacement of amino acids with similar structure or function is unlikely to disrupt the biological activity.

The "identity" of amino acid sequences refers to sequence similarity between two proteins or polypeptides. When a position in both of the two sequences to be compared is occupied by the same amino acid residue, e.g., if a position in both polypeptides is occupied by the same amino acid residue, the molecules are identical at that position. Examples of algorithms suitable for determining percent sequence identity and percent sequence similarity are the BLAST and BLAST 2.0 algorithms, which are described in Altschul et al. (1990) J. Mol. Biol. 215:403-410 and Altschul et al.(1977) Nucleic Acids Res. 25:3389-3402, respectively. Software for performing BLAST analyses is publicly available at the National Center for Biotechnology Information (http://www.ncbi.nlm.nih.gov/).

Methods for producing and purifying antibodies and antigen-binding fragments are well known in the prior art, for example, those described in chapters 5-8 and 15 of Antibodies: A Laboratory Manual, Cold Spring Harbor Press. The antibody or antigen-binding fragment described in the present invention is genetically engineered to contain one or more additional human-derived FRs in the non-human-derived CDRs. Human FR germline sequences can be obtained at the website http://imgt.cines.fr of ImMunoGeneTics (IMGT) or from the immunoglobulin journal, 2001ISBN012441351, by comparing the IMGT human antibody variable region germline gene database with the MOE software.

The engineered antibody or antigen-binding fragment of the present disclosure can be prepared and purified by conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems will result in glycosylation of antibodies, particularly at the highly conservative N-terminal site of the Fc region. Stable clones are obtained by expression of the antibody that specifically binds to human FXI. Positive clones are expanded in a serum-free medium of a bioreactor to produce antibodies. The culture medium with the secreted antibody can be purified by conventional techniques. For example, purification is performed using an A or G Sepharose FF column containing an adjusted buffer. Non-specifically bound fractions are washed away. The bound antibody is eluted by the pH gradient method, and the antibody fragments are detected by SDS-PAGE and collected. The antibody can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

"Administer" and "treat", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids. "Administer" and "treat" can refer to, for example, therapeutic, pharmacokinetic, diagnostic, research, and experimental methods. The treatment of cells comprises contacting the reagent with the cells and contacting the reagent with fluid, where the fluid is in contact with the cells. "Administer" and "treat" also refer to treating, e.g., cells, by reagents, diagnosis, binding compositions or by another cell *in vitro* and *ex vivo.* "Treat", when applied to humans, veterinary, or research subjects, refers to therapeutic treatment, preventive or prophylactic measures, and research and diagnostic applications. "Treatment" refers to administering a therapeutic agent, such as a composition comprising any one of the conjugation compounds of the present disclosure, either internally or externally to a patient with one or more symptoms of a disease on which the therapeutic agent is known to have a therapeutic effect. Typically, the therapeutic agent is administered in an amount effective to alleviate one or more symptoms of the disease in the patient or population being treated to induce regression of such symptoms or to inhibit the development of such symptoms to any clinically measurable degree. The amount of the therapeutic agent effective to alleviate any particular symptom of the disease (also known as a "therapeutically effective amount") may vary depending on a variety of factors, such as the disease state, age, and weight of the patient, and the ability of the drug to produce a desired therapeutic effect in the patient. Whether a disease symptom has been alleviated can be evaluated by any clinical testing methods commonly used by doctors or other health care professionals to evaluate the severity or progression of the symptom. Although the embodiments of the present disclosure (for example treatment methods or article of manufacture) may not be effective in alleviating all the target disease symptoms in every patient, as determined according to any statistical testing methods known in the art, such as Student t-test, chi-square test, Mann and Whitney's U test, Kruskal-Wallis test (H test), Jonckheere-Terpstra test, and Wilcoxon test, they should reduce the target disease symptoms in a statistically significant number of patients.

"Effective amount" comprises an amount sufficient to ameliorate or prevent a symptom or disorder of a medical disease. An effective amount also refers to an amount sufficient to allow or facilitate diagnosis. The effective amount for a particular patient or veterinary subject may vary depending on the factors such as the disorder to be treated, the general health of the patient, the method and route and dose of administration, and the severity of side effects. An effective amount may be the maximum dose or administration regimen to avoid significant side effects or toxic effects.

"Tm value" refers to the thermal denaturation temperature of a protein, i.e., the temperature at which half of the protein is unfolded and the spatial structure of the protein is destroyed. Therefore, the higher the Tm value, the higher the thermal stability of the protein.

### Examples

The present disclosure is further described below with reference to examples; however, these examples are not intended to limit the scope of the present disclosure. The experimental methods in the examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturer of the raw material or the goods. Reagents without specific origins indicated are commercially available conventional reagents.

### Example 1. Screening for and Preparation of Anti-FXI/FXIa Hybridoma Monoclonal Antibodies

### 1. Preparation of human FXI/FXIa antigen and detection protein

Human FXI/FXIa protein (Uniprot Acc No. P03951) was purchased from Enzyme research laboratories (FXI: Cat. HFXI1111; FXIa: HFXIa 1111a) as an antigen and a protein for detection involved in the present disclosure. Unless otherwise specified, the following FXI/FXIa antigen was human FXI/FXIa.

### > Amino acid sequence of human FXI/FXIa:

ECVTQLLKDTCFEGGDITTVFTPSAKYCQVVCTYHPRCLLFTFTAESPSEDPTRWFTCVLKD
SVTETLPRVNRTAAISGYSFKQCSHQISACNKDIYVDLDMKGINYNSSVAKSAQECQERCTD
DVHCHFFTYATRQFPSLEHRNICLLKHTQTGTPTRITKLDKVVSGFSLKSCALSNLACIRDIF
PNTVFADSNIDSVMAPDAFVCGRICTHHPGCLFFTFFSQEWPKESQRNLCLLKTSESGLPST
RIKKSKALSGFSLQSCRHSIPVFCHSSFYHDTDFLGEELDIVAAKSHEACQKLCTNAVRCQFF
TYTPAQASCNEGKGKCYLKLSSNGSPTKILHGRGGISGYTLRLCKMDNECTTKIKPRIVGGT
ASVRGEWPWQVTLHTTSPTQRHLCGGSIIGNQWILTAAHCFYGVESPKILRVYSGILNQSEI
KEDTSFFGVQEIIIHDQYKMAESGYDIALLKLETTVNYTDSQRPICLPSKGDRNVIYTDCWV
TGWGYRKLRDKIQNTLQKAKIPLVTNEECQKRYRGHKITHKMICAGYREGGKDACKGDSG
GPLSCKHNEVWHLVGITSWGEGCAQRERPGVYTNVVEYVDWILEKTQAV
(SEQ ID NO: 1)

Meanwhile, the following KLH-conjugated FXIa-specific polypeptides were synthesized for immunization of mice.
KLH- CFYGVESPKILRVYSGIL (SEQ ID NO: 2)
KLH- CGYRKLRDKIQNTLQKAKIPL (SEQ ID NO: 3)
KLH- CGVQEIIIHDQYKMAESGYDI (SEQ ID NO: 4)

### 2. Purification of FXI/FXIa-related recombinant protein and purification of hybridoma antibody and recombinant antibody

(1) Separation and purification/ProteinG affinity chromatography of mouse hybridoma supernatant:
   For the purification of mouse hybridoma supernatant, ProteinG packing material (KANEKA: Cat. KanCapTMG) was preferred for affinity chromatography. The cultured hybridoma was centrifuged to obtain the supernatant, which was then purified by a gravity column packed with ProteinG packing material. Firstly, the column was regenerated with 3-5 column volumes of 6 M guanidine hydrochloride (Simga: Cat. G3272-1KG) and then washed with 3-5 column volumes of pure water; the column was equilibrated with 3-5 column volumes of 1× PBS (pH 7.4) (Sangon Biotech (Shanghai) Co., Ltd.: Cat. E607016-0500) buffer system as an equilibration buffer; the cell supernatant was loaded on the column at a low flow rate for binding, with the flow rate controlled to allow for retention time of about 1 min or longer; the column was washed with 3-5 column volumes of 1× PBS (pH 7.4) until the UV absorbance fell back to baseline; the sample was eluted with a 0.1 M Glycine (pH 3.0) (Sigma: Cat. 410225-250G) buffer, the elution peaks were collected based on UV detection, and the eluted product was rapidly adjusted to pH 7-8 with 1 M Tris-HCl (pH 8.0) (Vetec, Cat. V900483) and temporarily stored. For the eluted product, solution exchange may be performed by methods well known to those skilled in the art, such as ultrafiltration concentration using an ultrafiltration tube to replace the solution with a desired buffer system, or replacement with a desired buffer system by size exclusion (e.g., G-25 desalination).
(2) Extraction of the human Fc-tagged fusion protein or antibody by Protein A affinity chromatography:
   Firstly, the cell culture expressing the Fc fusion protein or antibody was centrifuged at a high speed to obtain the supernatant. A Protein A (GE, Cat: 17-5474-99) affinity column was regenerated with 5 column volumes of 0.1 M NaOH (Sigma: Cat: 71687-500g) and washed and equilibrated with 5 column volumes of 1× PBS (pH 7.4) (Sangon Biotech (Shanghai) Co., Ltd.: Cat. E607016-0500). The cell supernatant was loaded on the column at a low flow rate for binding, with the flow rate controlled to allow for retention time of about 1 min or longer. After the binding was completed, the column was washed with 5 column volumes of 1× PBS (pH 7.4) until the UV absorbance fell back to baseline. The sample was eluted with a 0.1 M Glycine (pH 3.0) (Sigma: Cat. 410225-250G) buffer, the elution peaks were collected based on UV detection, and the eluted product was rapidly adjusted to pH 7-8 with 1 M Tris-HCl (pH 8.0) (Vetec, Cat. V900483) and temporarily stored. For the eluted product, solution exchange may be performed by methods well known to those skilled in the art, such as ultrafiltration concentration using an ultrafiltration tube to replace the solution with a desired buffer system, or replacement with a desired buffer system by size exclusion (e.g., G-25 desalination).
(3) Polishing purification by anion chromatography:
   Firstly, the sample was diluted with an equilibration buffer (20 mM Tris pH 8.0 (Vetec, Cat. V900483)) to allow the conductivity to be less than 3 ms/cm, and the Q HP (GE, Cat: 17-1014-01) anion chromatography column was regenerated with 5 column volumes of 0.5 M NaOH (Sigma, Cat: 71687-500g), and washed and equilibrated with 15 column volumes of 20 mM Tris pH 8.0 (Vetec, Cat. V900483). The sample was loaded at a low flow rate for binding, with the flow rate controlled to allow for retention time of about 2 min or longer. After the binding was completed, the column was washed with 10 column volumes of 20 mM Tris pH 8.0 (Vetec, Cat. V900483) until the UV absorbance fell back to baseline. 0-100% gradient elution was performed with an elution buffer (20 mM Tris pH 8.0 (Vetec, Cat. V900483)) and 0.5 M NaCl (Vetec, Cat. V900058); elution peaks were collected according to SEC-UPLC (Column: Waters ACQUITY UPLC^{®} Protein BEH SEC column 200Å, 1.7 µm, Cat. 186005225) purity determination. For the eluted product, solution exchange may be performed by methods well known to those skilled in the art, such as ultrafiltration concentration using an ultrafiltration tube to replace the solution with a desired buffer system, or replacement with a desired buffer system by size exclusion (e.g., G-25 desalination).

### 3. Antibody screening

Five SJL white mice and 5 Balb/c white mice were each immunized with 25 µg of a mixture of FXIa antigen, 3 KLH-conjugated polypeptide, and an adjuvant. The immunization was performed on day 0, day 14, and day 35. On day 0, 25 µg of emulsified antigen was injected intraperitoneally (IP) into each mouse. On day 14 and day 35, 12.5 µg of emulsified antigen was injected into each mouse. Blood was collected on day 21 and day 42, and the antibody titer in mouse serum was determined by ELISA. After 4-5 immunizations, mice in which the antibody titer in serum was high and was reaching a plateau were selected for splenocyte fusion.

Spleen lymphocytes and myeloma cells Sp2/0-Ag14 were fused by following an optimized PEG-mediated fusion procedure to obtain hybridoma cells. Based on the growth density of hybridoma cells, hybridoma culture supernatants were subjected to purification, a cell binding experiment, and a cell blocking experiment by using a binding ELISA, and cells in the positive wells were promptly expanded, frozen, conserved, and sequenced. The ELISA method was used for further screening of hybridoma cells to obtain hybridoma clones, and then the antibody was prepared and purified.

A positive clone 3807 was obtained. The corresponding amino acid sequences of variable regions of the antibody are as follows:
>3807-VH: 
EVQLQQSGAELVRPGTSVKLSCIASGFNIKDDYMHWVKQRPEQGLEWIGWIDPENGDTEY
ASKFQGKATITADTSANTAYLQFSSLTSEDTAVYYCLYGNFYYFDYWGQGTTLTVSS
SEQ ID NO: 5
>3807-VL:

QIVLTQSPAIMSASPGEKVTMTCSASSSINYMHWYQQKPGTSPKRWIYDTSKLASGVPARFS
GSGSGTSYSLTISSMEAEDAATYYCHQRSFSPLTFGAGTKLELK
SEQ ID NO: 6

**Table 1. CDR sequences of heavy and light chains of antibody 3807**

| Heavy chain variable region (VH) | | Light chain variable region (VL) | |
|---|---|---|---|
| HCDR1 | DDYMH (SEQ ID NO: 7) | LCDR1 | SASSSINYMH (SEQ ID NO: 10) |
| HCDR2 | WIDPENGDTEYASKFQG (SEQ ID NO: 8) | LCDR2 | DTSKLAS (SEQ ID NO: 11) |
| HCDR3 | GNFYYFDY (SEQ ID NO: 9) | LCDR3 | HQRSFSPLT (SEQ ID NO: 12) |

### Example 2. Humanization and Immunogenicity Engineering of Anti-FXI/FXIa Hybridoma Antibody

### 1. Humanization

By three-dimensional structure homologous modeling of the antibody molecule 3807, in combination with the results of comparison with a V-base human germline sequence database and an IMGT human antibody heavy chain variable region germline gene database, heavy chain variable region germline genes with high homology with the selected antibody were selected as templates, and CDRs of the mouse-derived monoclonal antibody were grafted into corresponding human-derived moieties. Three-dimensional structure modeling and analysis were performed on the grafted antibody, and back mutations were performed on the specific sites in the FR regions that affected the structure and morphology of the CDR regions. The amino acid residues were identified using the Kabat numbering scheme and annotated.

For the humanization framework of the hybridoma clone 3807, the light chain template was IGKV3-11*01, and the heavy chain template was IGHV1-69-2*01. The humanized variable region sequences are as follows (with the underlined portions being CDRs):
>3807VH-CDR graft 
QVQLVQSGAEVKKPGSSVKVSCKASGYTFTDDYMHWVRQAPGQGLEWMGWIDPENGDT
EYASKFQGRVTITADESTSTAYMELSSLRSEDTAVYYCARGNFYYFDYWGQGTTVTVSS
SEQ ID NO: 13
>3807VL-CDR graft

EIVLTQSPATLSLSPGERATLSCSASSSINYMHWYQQKPGQAPRLLIYDTSKLASGIPARFSGS
GSGTDFTLTISSLEPEDFAVYYCHQRSFSPLTFGQGTKLEIK
SEQ ID NO: 14

The back mutation sites for hybridoma clone 3807 and the design of the mode of mutation are shown in Table 2.

**Table 2. Sequences of each of the heavy chain and light chain variable regions**

| 3807-VH | |
|---|---|
| 3807-VH1 | Y27F, T28N, F29I, T30K, A93L, R94Y |
| 3807-VH2 | Y27F, T28N, F29I, T30K, A93L, R94Y, E73T |
| 3807-VH3 | Y27F, T28N, F29I, T30K, A93L, R94Y, E73T, V67A |
| 3807-VH4 | Y27F, T28N, F29I, T30K, A93L, R94Y, E73T, R66K, V67A |
| 3807-VH5 | Y27F, T28N, F29I, T30K, A93L, R94Y, E73T, T75A, T76N |

| 3807-VL | |
|---|---|
| 3807-VL1 | No back mutation |
| 3807-VL2 | L47W, I58V |
| 3807-VL3 | R45K, L46R, L47W, I58V |
| 3807-VL4 | L47W, I58V, F71Y |

Wherein, the specific sequences of 3807-VH1 and 3807-VL3 are as follows.
>3807-VH1: 
QVQLVQSGAEVKKPGSSVKVSCKASGFNIKDDYMHWVRQAPGQGLEWMGWIDPENGDT
EYASKFQGRVTITADESTSTAYMELSSLRSEDTAVYYCLYGNFYYFDYWGQGTTVTVSS
SEQ ID NO: 15
>3807-VL3:

EIVLTQSPATLSLSPGERATLSCSASSSINYMHWYQQKPGQAPKRWIYDTSKLASGVPARFSG
SGSGTDFTLTISSLEPEDFAVYYCHQRSFSPLTFGQGTKLEIK
SEQ ID NO: 16

For the antibody 3871, the VH was 3807-VH1 and the VL was 3807-VL3.

### 2. Immunogenicity engineering

3871 was genetically engineered to provide an antibody with higher stability and lower immunogenicity. Only the FR regions of the heavy chain variable region were engineered, with the light chain variable region unchanged.

The sequence of the genetically engineered heavy chain variable region of the humanized antibody 3871 was as follows (light chain unchanged):

### >3807-VH1(GTFS)

QVQLVQSGAEVKKPGSSVKVSCKASGGTFSDDYMHWVRQAPGQGLEWMGWIDPENGDT
EYASKFQGRVTITADESTSTAYMELSSLRSEDTAVYYCLYGNFYYFDYWGQGTTVTVSS
SEQ ID NO: 17

For the antibody 3882, the VH was 3807-VH1 (GTFS) and the VL was 3807-VL3.

Each of the heavy chain variable regions was fused with the heavy chain CH1 (SEQ ID NO: 18) of the corresponding human-derived antibody and the Fc (containing the S241P mutation) (SEQ ID NO: 19) of the human antibody IgG4, and the light chain variable region was fused with the human κ (SEQ ID NO: 20), thus forming a recombinant chimeric antibody for subsequent assay.
> Heavy chain CH1 of human antibody: 
ASTKGPSVFPLAPCSRSTSESTAALGCLVKDYFPEPVTVSWNSGALTSGVHTFPAVLQSSGLY
SLSSVVTVPSSSLGTKTYTCNVDHKPSNTKVDKRV
SEQ ID NO: 18
> IgG4PFc (i.e., containing the S241P mutation) of human antibody: 
ESKYGPPCPPCPAPEFLGGPSVFLFPPKPKDTLMISRTPEVTCVVVDVSQEDPEVQFNWYVD
GVEVHNAKTKPREEQFNSTYRVVSVLTVLHQDWLNGKEYKCKVSNKGLPSSIEKTISKAK
GQPREPQVYTLPPSQEEMTKNQVSLTCLVKGFYPSDIAVEWESNGQPENNYKTTPPVLDSD
GSFFLYSRLTVDKSRWQEGNVFSCSVMHEALHNHYTQKSLSLSLGK
SEQ ID NO: 19
> light chain Cκ of human antibody:



SEQ ID NO: 20

Full length sequences of the antibody exemplified with 3882:
>3882-HC: 
QVQLVQSGAEVKKPGSSVKVSCKASGGTFSDDYMHWVRQAPGQGLEWMGWIDPENGDT
EYASKFQGRVTITADESTSTAYMELSSLRSEDTAVYYCLYGNFYYFDYWGQGTTVTVSSAST
KGPSVFPLAPCSRSTSESTAALGCLVKDYFPEPVTVSWNSGALTSGVHTFPAVLQSSGLYSLS
SVVTVPSSSLGTKTYTCNVDHKPSNTKVDKRVESKYGPPCPPCPAPEFLGGPSVFLFPPKPK
DTLMISRTPEVTCVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPSQEEMTKNQVSLTCLV
KGFYPSDIAVEWESNGQPENNYKTTPPVLDSDGSFFLYSRLTVDKSRWQEGNVFSCSVMHE
ALHNHYTQKSLSLSLGK
SEQ ID NO: 21
>3882-LC:

EIVLTQSPATLSLSPGERATLSCSASSSINYMHWYQQKPGQAPKRWIYDTSKLASGVPARFSG
SGSGTDFTLTISSLEPEDFAVYYCHQRSFSPLTFGQGTKLEIKRTVAAPSVFIFPPSDEQLKSGT
ASVVCLLNNFYPREAKVQWKVDNALQSGNSQESVTEQDSKDSTYSLSSTLTLSKADYEKH
KVYACEVTHQGLSSPVTKSFNRGEC
SEQ ID NO: 22

After preparation of an expression vector of the antibody, transfection of CHO cells, separation, purification, and detection by conventional methods, the antibodies of interest were obtained.

### Example 3. Functional Validation of Anti-FXI/FXIa Antibodies

### 1. Affinity assay

The Biacore method was used. A certain amount of test antibody was subjected to affinity capture by a Protein A biosensor chip (Cat. # 29127556, GE), and then human FXI/FXIa at a series of concentration gradients were allowed to flow on the surface of the chip. The reaction signals were detected in real time by using a Biacore instrument (Biacore T200, GE) to obtain association and dissociation curves. After dissociation was completed for each cycle, the biochip was washed and regenerated with a regeneration solution provided in a human antibody capture kit or a glycine-hydrochloric acid regeneration solution (pH 1.5, Cat. # BR-1003-54, GE). The buffer used in the experiment was HBS-EP + 10× buffer solution (Cat. # BR-1006-69, GE) diluted to 1× (pH 7.4) with D. I. Water.

The data obtained from the experiment were fitted with the (1:1) Langmuir model using BIAevaluation version 4.1 software (GE) to obtain affinity values. The test results are shown in Table 3.

**Table 3. Affinity determination of anti-FXI/FXIa antibodies**

| Antibody | Antigen | ka (1/Ms) | kd (1/s) | KD (M) |
|---|---|---|---|---|
| 3871 | FXI | 1.32E+06 | 2.66E-05 | 2.00E-11 |
| 3882 | FXI | 1.973E+6 | 5.846E-5 | 2.963E-11 |
| 3807 | FXIa | 1.59E+06 | 2.35E-04 | 1.48E-10 |
| 3871 | FXIa | 1.24E+06 | 7.08E-04 | 5.70E-10 |
| 3882 | FXIa | 1.06E+06 | 3.87E-04 | 3.64E-10 |

### 2. Experiment on inhibition of FXIIa-mediated FXI activating enzyme activity in vitro

The ability of the binding of the anti-FXI/FXIa antibody to trypsinogen FXI to block the cleavage of FXI by FXIIa was tested.

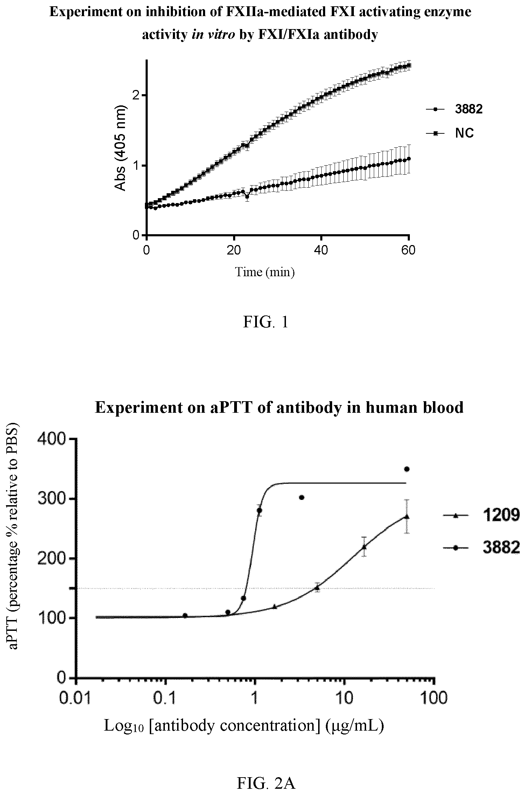

A SpectraMax M5 microplate reader was preset to 37 °C. A 384-well plate was pre-cooled on ice, followed by the addition of 10 µL of FXI (12 µg/mL), 10 µL of FXIIa (7.8 µg/mL), 10 µL of a test FXI/FXIa antibody (300 µg/mL), and 10 µL of Dextran (100 µg/mL) each diluted with a buffer (20 mM HEPES, pH 7.4, 150 mM NaCl and 0.1% BSA); the plate was incubated at 37 °C for 60 min and then incubated on ice for 5 min; after 10 µL of S-2366 (8 mM) (Chromogenix, S821090) was added, the plate was read using the microplate reader. The results are shown in FIG. 1, wherein human IgG isotype was used as a negative control (NC).

### 3. Determination of aPTT and PT anticoagulant activity in human/monkey blood

Human/monkey blood was collected freshly using sodium citrate tubes and centrifuged at 3000 rpm for 15 min, and the upper layer plasma was collected.

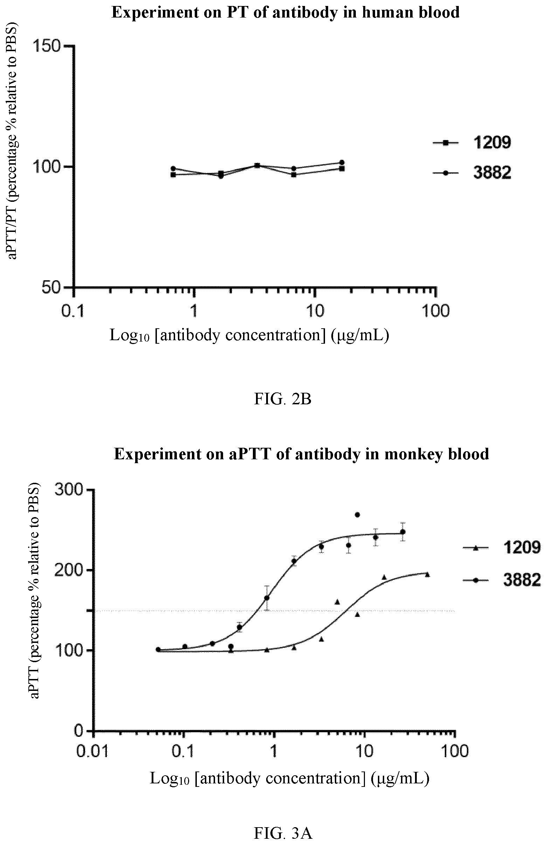

Activated partial thromboplastin time (aPTT) was determined with the Sysmex kit in the presence of the candidate antibody at different concentrations (diluted with PBS), and the candidate antibody to be tested was incubated with plasma at 37 °C for 3 min. Coagulation was then initiated by the addition of 25 mM calcium chloride reagent and the time when the coagulation occurred was determined. The concentration of candidate antibody that prolonged the aPTT by 50% (EC50) was determined. The test results of part of the antibodies are shown in FIG. 2A, FIG. 3A, and Table 4. 1209 was another anti-FXI/FXIa antibody obtained by screening in the present application.

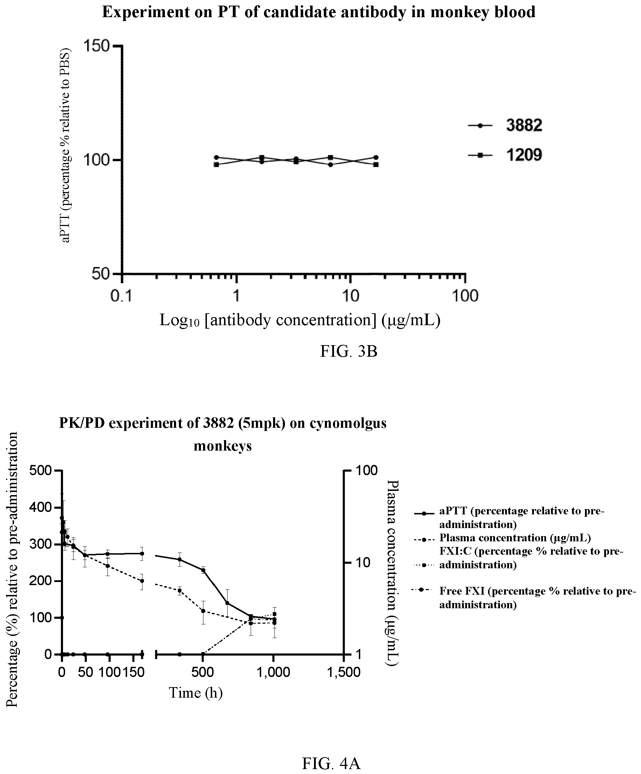

Prothrombin time (PT) was determined with the Sysmex kit in the presence of the candidate antibody at different concentrations (diluted with PBS), and the candidate antibody to be tested was incubated with plasma at 37 °C for 3 min. Coagulation was then initiated by the addition of thromboplastin and the time when the coagulation occurred was determined. The test results of part of the antibodies are shown in FIG. 2B and FIG. 3B.

**Table 4. Determination of aPTT/PT of anti-FXI/FXIa antibodies**

| Antibody | Plasma | aPTT1.5 (ug/mL) | EC₅₀ (ug/mL) |
|---|---|---|---|
| 3882 | Human blood | 0.91 | 1.24 |
| 3882 | Monkey blood | 0.98 | 1.25 |

### 4. Pharmacokinetic (PK)/pharmacodynamic (PD) assay in cynomolgus monkeys

Normal adult male cynomolgus monkeys (body weight in the range of 4.0-4.7 kg) were grouped according to body weight (monkeys weighing 1st, 4th, and 5th were assigned to one group, and monkeys weighing 2nd, 3rd, and 6th were assigned to the other group) with 3 animals in each group and observed for 14 days.

Blood samples were collected one day before administration using sodium citrate blood collection tubes and, as the samples before administration, were determined for aPTT, PT, plasma drug concentration, plasma active XI factor (Factor XI, FXI) ratio (FXI:C%, i.e., the proportion of FXI with coagulation activity), and plasma free FXI concentration; and bleeding time was also determined for each animal. Of the two groups of animals, one group was blank group and was not subjected to compound administration, and the other group was given a compound at 3882, 5 mg/kg body weight (mg/kg, mpk). The compound was dissolved in phosphate buffer (PBS) at a concentration of 5 mg/mL and administered by intravenous injection.

The blank control group was observed for bleeding time 15 minutes (min) and 3 hours (h) after the administration; plasma was collected and observed for aPTT and PT 15 min, 3 h, 6 h, and 1 day (d) after the administration according to the method described above. The administration group was observed for bleeding time 15 min, 3 h, 2 d, 4 d, 1 week (w), 2 w, and 3 w after the administration; plasma was collected and observed for aPTT, PT, plasma drug concentration, plasma FXI:C%, and plasma free FXI concentration 15 min, 3 h, 6 h, 1 d, 2 d, 4 d, 1 w, 2 w, 3 w, 4 w, 5 w, and 6 w after the administration according to the method described above. AV-shunt thrombus construction was performed on both groups of animals 1 d after the administration, wherein the thrombus construction time was 10 min, and the net weight of the thrombus was weighed after thrombus construction. aPTT, PT, and plasma FXI:C% were determined by a coagulometer and corresponding kits, and plasma free FXI concentration and plasma drug concentration were determined by an ELISA method. The thrombus weight of the administration group was compared with that of the control group, and statistical analysis was performed by using t-test.

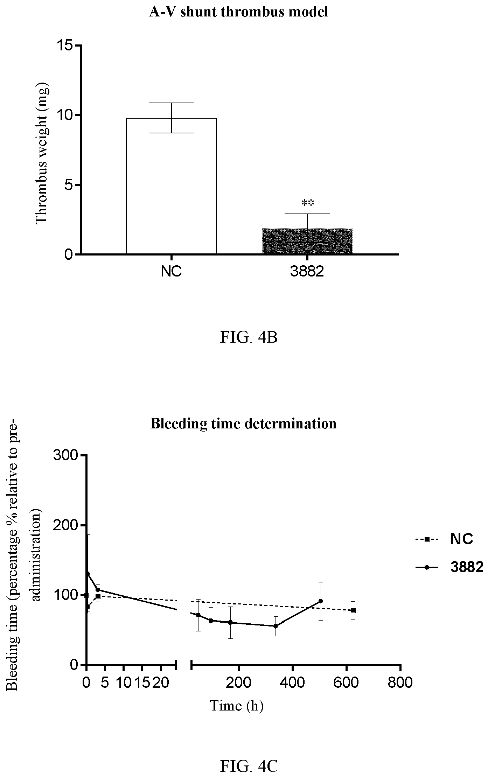

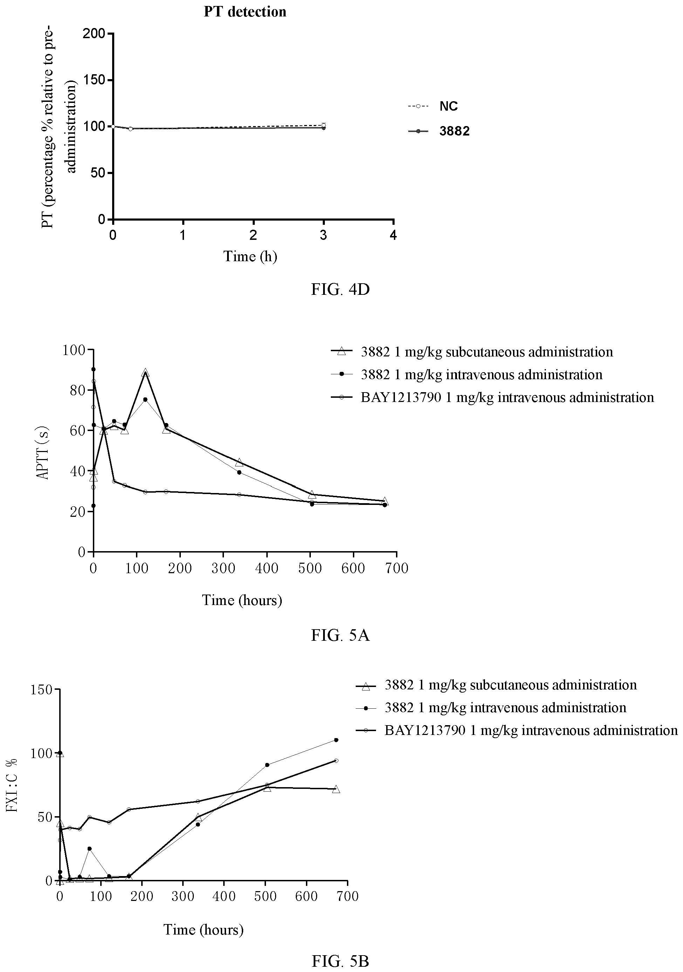

The test results of the antibodies are shown in FIGs. 4A-4D, wherein the negative control (NC) indicated no compound administered. The results showed that 5 mpk well inhibited thrombogenesis and prolonged the endogenous coagulation time, but had no significant influence on the bleeding time and the exogenous coagulation time of animals, and the PK results showed that the half-life period was about 20 days.

### 5. Pharmacodynamic (PD) experiment in cynomolgus monkeys

Six Normal adult male cynomolgus monkeys (body weight in the range of 7-9 kg) were randomly divided into 3 groups, which were: BAY1213790 1 mg/kg intravenous administration group, 3882 1 mg/kg intravenous administration group, and 3882 1 mg/kg subcutaneous administration group. The blood samples of animals in the intravenous administration groups were collected before the administration and 5 min, 1 h, 1 d, 2 d, 3 d, 5 d, 1 w, 2 w, 3 w, and 4 w after the administration, the blood samples of animals in the subcutaneous administration group were collected before the administration and 1 h, 1 d, 2 d, 3 d, 5 d, 1 w, 2 w, 3 w, and 4 w after the administration, all with sodium citrate blood collection tubes, and aPTT and plasma FXI:C% were determined with a coagulometer and corresponding kits.

The test results are shown in FIG. 5A and FIG. 5B. The results showed that 3882 was able to significantly prolong the endogenous coagulation time and inhibit the activity of FXI by both subcutaneous administration and intravenous administration. 3882 exhibited longer maintenance time for prolonging the endogenous coagulation time and stronger inhibition effect on FXI compared with BAY1213790 when administered at an equal dose.
> Heavy chain of BAY1213790: EVQLLESGGGLVQPGGSLRLSCAASGFTFSQYGMDWVRQAPGKGLEWVSGIGPSGGSTVY 
ADSVKGRFTISRDNSKNTLYLQMNSLRAEDTAVYYCTRGGPYYYYGMDVWGQGTTVTVS
SASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTKN
QVSLTCLVKGFYPSDIAVEWESNGQPENNYKTTPPVLDSDGSFFLYSKLTVDKSRWQQGNV
FSCSVMHEALHNHYTQKSLSLSPG (SEQ ID NO: 23)
> Light chain of BAY1213790:

DIQMTQSPSSLSASVGDRVTITCQASQDISNYLNWYQQKPGKAPKLLIYDASNLETGVPSRF
SGSGSGTDFTFTISSLQPEDIATYYCQQADSFPVTFGGGTKVEIKRTVAAPSVFIFPPSDEQLK
SGTASVVCLLNNFYPREAKVQWKVDNALQSGNSQESVTEQDSKDSTYSLSSTLTLSKADY
EKHKVYACEVTHQGLSSPVTKSFNRGEC          (SEQ ID NO: 24)

The anti-FXI/FXIa antibody in Examples 4-9 was 3882, the heavy chain sequence and light chain sequence of which are set forth in SEQ ID NO: 21 and SEQ ID NO: 22, respectively.

### Example 4. Screening for pH of Buffer System in Anti-FXI/FXIa Antibody Formulation

The buffers in Table 5 were formulated to prepare antibody formulations in which the anti-FXI/FXIa antibody had a concentration of 120 mg/mL, and samples were taken for stability examination under high temperature of 40 °C, shaking, and freeze-thawing.

**Table 5. Results of screening for pH/buffer in anti-FXI/FXIa antibody**

| Buffer system | Conditions | Appearance | SEC % | NRCE % | iCIEF % | | |
|---|---|---|---|---|---|---|---|
| | | | Main peak | Main peak | Acidic peak | Main peak | Basic peak |
| 10 mM acetic acid-sodium acetate pH 4.5 (No: F1-4.5) | T0 | C, CL, particle-free | 97.6 | 97.8 | 18.4 | 68.6 | 13.0 |
| | Shaking for 5D | C, WO, particle-free | 96.7 | 97.5 | 19.0 | 68.5 | 12.5 |
| | 5 cycles of freeze-thawing | C, CL, P+ | 96.3 | 97.8 | 18.3 | 67.4 | 14.4 |
| | 40°C-15D | C, WO, P++ | 91.5 | 96.9 | 19.1 | 63.2 | 17.7 |
| 10 mM acetic acid-sodium acetate pH 5.0 (No.: F2-5.0) | T0 | C, SO, particle-free | 97.1 | 97.8 | 19.3 | 67.1 | 13.6 |
| | Shaking for 5D | C, SO, particle-free | 96.9 | 97.8 | 19.0 | 68.2 | 12.8 |
| | 5 cycles of freeze-thawing | C, SO, P+ | 96.6 | 97.9 | 20.0 | 66.4 | 13.7 |
| | 40°C-15D | C, SO, P+++ | 93.9 | 97.9 | 21.9 | 62.9 | 15.3 |
| 10 mM histidine-histidine hydrochloride pH 6.0 (No.: F3-6.0) | T0 | C, SO, particle-free | 98.4 | 97.8 | 19.0 | 67.9 | 13.1 |
| | Shaking for 5D | C, SO, particle-free | 97.5 | 97.8 | 18.7 | 68.5 | 12.9 |
| | 5 cycles of freeze-thawing | C, SO, P+++ | 97.2 | 97.7 | 19.1 | 67.9 | 13.0 |
| | 40°C-15D | C, SO, P+++ | 96.1 | 97.3 | 25.5 | 63.2 | 11.3 |
| 10 mM histidine-histidine hydrochloride pH 6.5 (No.: F4-6.5) | T0 | C, CL, particle-free | 97.7 | 97.7 | 18.6 | 68.3 | 13.2 |
| | Shaking for 5D | C, WO, particle-free | 97.0 | 97.9 | 19.6 | 67.3 | 13.1 |
| | 5 cycles of freeze-thawing | C, CL, P+ | 96.7 | 97.8 | 19.6 | 66.9 | 13.5 |
| | 40°C-15D | C, WO, P++ | 96.1 | 97.4 | 26.0 | 63.0 | 11.0 |
| 10 mM sodium dihydrogen phosphate-disodium hydrogen phosphate pH 7.0 (No.: F5-7.0) | T0 | C, CL, particle-free | 97.5 | 97.6 | 19.0 | 67.9 | 13.1 |
| | Shaking for 5D | C, WO, particle-free | 96.5 | 97.8 | 19.5 | 67.7 | 12.8 |
| | 5 cycles of freeze-thawing | C, CL, P+ | 96.0 | 97.7 | 19.4 | 67.8 | 12.8 |
| | 40°C-15D | C, WO, P++ | 95.5 | 97.0 | 33.9 | 55.9 | 10.2 |
| 10 mM Tris-hydrochloric acid, pH 7.5 (No.: F6-7.5) | T0 | C, SO, particle-free | 97.7 | 97.5 | 19.3 | 67.6 | 13.1 |
| | Shaking for 5D | C, SO, particle-free | 96.8 | 97.7 | 18.7 | 69.1 | 12.2 |
| | 5 cycles of freeze-thawing | C, SO, P++ | 96.3 | 97.7 | 19.6 | 67.1 | 13.3 |
| | 40°C-15D | C, SO, P+++ | 95.4 | 97.7 | 25.6 | 62.9 | 11.5 |
| 10 mM Tris-hydrochloric acid, pH 8.0 (No.: F7-8.0) | T0 | C, CL, particle-free | 97.3 | 97.8 | 19.2 | 67.8 | 13.0 |
| | Shaking for 5D | C, CL, particle-free | 96.5 | 97.9 | 20.9 | 65.6 | 13.5 |
| | 5 cycles of freeze-thawing | C, CL, P+++ | 96.1 | 97.7 | 20.4 | 66.4 | 13.3 |
| | 40°C-15D | C, CL, P+++ | 95.8 | 95.7 | 43.4 | 46.6 | 10.0 |
| 10 mM Tris-hydrochloric acid, pH 8.5 (No.: F8-8.5) | T0 | C, CL, particle-free | 97.4 | 97.7 | 19.7 | 66.7 | 13.7 |
| | Shaking for 5D | C, CL, particle-free | 96.5 | 97.5 | 22.5 | 65.5 | 12.0 |
| | 5 cycles of freeze-thawing | C, CL, P++ | 96.0 | 97.5 | 20.4 | 66.7 | 12.9 |
| | 40°C-15D | C, CL, P+++ | 94.6 | 94.8 | 53.8 | 37.6 | 8.6 |

| | | | | | | | |
|---|---|---|---|---|---|---|---|
| Note: C = colorless; CL = clear; WO = micro-opalescent; SO = strongly opalescent; P+ (a small number of particles); P++ (many particles); P+++ (a large number of particles) | | | | | | | |

The appearance results showed that: no formulas showed generation of particles after shaking for 5D; all formulas showed generation of particles after 5 cycles of freeze-thawing, with relatively less particles generated by the 10 mM acetic acid-sodium acetate pH 4.5 and pH5.0 systems, the 10 mM histidine-histidine hydrochloride pH 6.5 system, and the 10 mM sodium dihydrogen phosphate-disodium hydrogen phosphate pH 7.0 system; all formulas showed generation of particles under conditions of 40 °C-15D, with less particles generated by the 10 mM histidine-histidine hydrochloride pH 6.5 and 10 mM sodium dihydrogen phosphate-disodium hydrogen phosphate pH 7.0 than by the other two systems.

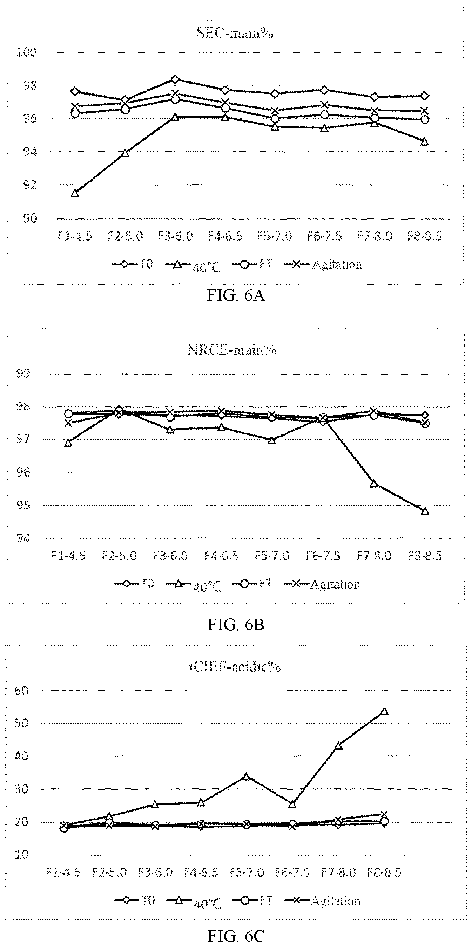

The SEC (FIG. 6A) results showed that: all formulas showed increase of aggregates under conditions of 40 °C-15D; under conditions of 40 °C-15D, SEC aggregates decreased significantly with the increase of pH when the pH was in the range of 4.5-6.5; under conditions of 40 °C-15D, SEC aggregates increased significantly when the pH increased from 8.0 to 8.5; when the pH was between 6.0 and 8.0, the 40 °C-15D SEC results showed <2.5% increase of aggregates compared with T0, which was within an acceptable range.

NRCE (FIG. 6B) results showed that: the purity was reduced under conditions of 40 °C-15D; in the 10 mM Tris-hydrochloric acid system, the NRCE purity was significantly reduced with the increase of pH (pH 7.5-8.5), and in other buffer systems with the pH ranging from 4.5 to 7.5, the NRCE purity was not significantly different.

The iCIEF (FIG. 6C) results showed that: within the same buffer system, the acidic peak increased significantly with the increase of pH.

Taking the appearance, SEC, NRCE, and iCIEF results into consideration, the pH 6.5-7.5 buffer systems (histidine-histidine hydrochloride, sodium dihydrogen phosphate-disodium hydrogen phosphate, and Tris-hydrochloric acid systems) were suitable.

### Example 5. Screening for pH, Auxiliary Material, and Surfactant Concentration in Anti-FXI/FXIa Antibody formulation

Within the pH range of 6.5-7.5, 10 mM histidine-histidine hydrochloride, 10 mM sodium dihydrogen phosphate-disodium hydrogen phosphate, 10 mM Tris-citric acid, and 10 mM Tris-hydrochloric acid buffer systems were selected to prepare formulations containing 120 mg/mL anti-FXI/FXIa antibody, different kinds of auxiliary materials, and different kinds of surfactants. The samples were placed in an incubator at 40±2 °C and 75±5% RH for stability examination. The results showed that the 3882 antibody was sensitive to ionic strength and the stability of the antibody was low and particles were easily formed at high ionic strength.

The 10 mM histidine-histidine hydrochloride pH 6.5 and 7.0 buffer systems and 10 mM Tris-hydrochloric acid pH 7.0 and 7.5 buffer systems were selected to prepare formulations containing 60 mg/mL anti-FXI/FXIa antibody, 0.4-0.8 mg/mL polysorbate 80, and the auxiliary material, i.e., 80 mg/mL sucrose or 100 mM glycine + 40 mg/mL sucrose or 100 mM proline +40 mg/mL sucrose, and the formulations were subjected to stability examination under high temperature of 40 °C, shaking, and freeze-thawing:
F2) 10 mM histidine-histidine hydrochloride pH 6.5, 0.4 mg/mL polysorbate 80, 80 mg/mL sucrose;
F3) 10 mM histidine-histidine hydrochloride pH 6.5, 0.6 mg/mL polysorbate 80, 80 mg/mL sucrose;
F4) 10 mM histidine-histidine hydrochloride pH 6.5, 0.8 mg/mL polysorbate 80, 80 mg/mL sucrose;
F5) 10 mM histidine-histidine hydrochloride pH 6.5, 0.4 mg/mL polysorbate 80, 100 mM glycine + 40 mg/mL sucrose;
F6) 10 mM histidine-histidine hydrochloride pH 6.5, 0.4 mg/mL polysorbate 80, 100 mM proline + 40 mg/mL sucrose;
F7) 10 mM histidine-histidine hydrochloride pH 7.0, 0.4 mg/mL polysorbate 80, 80 mg/mL sucrose;
F8) 10 mM histidine-histidine hydrochloride pH 7.0, 0.4 mg/mL polysorbate 80, 100 mM proline + 40 mg/mL sucrose;
F10) 10 mM Tris-hydrochloric acid pH 7.0, 0.4 mg/mL polysorbate 80, 80 mg/mL sucrose;
F11) 10 mM Tris-hydrochloric acid pH 7.5, 0.4 mg/mL polysorbate 80, 80 mg/mL sucrose;

**Table 7. Results of fine-screening for pH, auxiliary material, and surfactant concentration in anti-FXI/FXIa antibody**

| No. | Conditions | Actual pH | Appearance | SEC % | NRCE % | CEX % | | |
|---|---|---|---|---|---|---|---|---|
| | | | | Main peak | Main peak | Acidic peak | Main peak | Basic peak |
| F2 | T0 | 6.50 | C, CL, particle-free | 99.2 | 96.8 | 7.6 | 51.4 | 41.0 |
| | Shaking for 3D | | C, CL, particle-free | 99.2 | 96.5 | 7.8 | 51.5 | 40.7 |
| | 5 cycles of freeze-thawing | | C, CL, P+++ | 99.2 | 96.6 | 7.4 | 53.0 | 39.5 |
| | 40°C-2W | | C, WO, P+ | 98.8 | 96.4 | 11.9 | 51.4 | 36.7 |
| | 40°C-1M | | C, WO, P+++ | 97.1 | 95.1 | 19.8 | 49.6 | 30.5 |
| F3 | T0 | 6.51 | C, CL, particle-free | 99.3 | 96.6 | 7.6 | 51.5 | 40.9 |
| | Shaking for 3D | | C, CL, particle-free | 99.2 | 96.1 | 7.8 | 51.6 | 40.6 |
| | 5 cycles of freeze-thawing | | C, CL, P+++ | 99.2 | 96.2 | 7.5 | 53.0 | 39.6 |
| | 40°C-2W | | C, WO, P+++ | 98.8 | 96.4 | 11.7 | 51.6 | 36.7 |
| | 40°C-1M | | C, WO, P+++ | 97.3 | 94.9 | 19.3 | 49.8 | 31.0 |
| F4 | T0 | 6.50 | C, CL, particle-free | 99.2 | 96.7 | 7.7 | 51.4 | 40.9 |
| | Shaking for 3D | | C, CL, P+++ | 99.2 | 96.7 | 7.8 | 51.7 | 40.5 |
| | 5 cycles of freeze-thawing | | C, CL, P+++ | 99.2 | 96.3 | 7.8 | 51.4 | 40.8 |
| | 40°C-2W | | C, WO, P+++ | 98.8 | 96.5 | 11.8 | 51.9 | 36.3 |
| | 40°C-1M | | C, WO, P+++ | 97.3 | 94.2 | 19.0 | 50.2 | 30.8 |
| F5 | T0 | 6.49 | C, CL, particle-free | 99.2 | 96.1 | 7.8 | 51.5 | 40.8 |
| | Shaking for 3D | | C, CL, particle-free | 99.2 | 96.8 | 7.8 | 51.5 | 40.7 |
| | 5 cycles of freeze-thawing | | C, CL, P+++ | 99.2 | 96.6 | 7.3 | 53.3 | 39.4 |
| | 40°C-2W | | C, WO, P+++ | 98.9 | 96.1 | 12.2 | 51.6 | 36.2 |
| | 40°C-1M | | C, WO, P+++ | 97.0 | 94.6 | 20.6 | 49.7 | 29.7 |
| F6 | T0 | 6.54 | C, CL, particle-free | 99.2 | 96.8 | 7.1 | 53.3 | 39.6 |
| | Shaking for 3D | | C, CL, P+++ | 99.2 | 96.9 | 7.7 | 51.6 | 40.7 |
| | 5 cycles of freeze-thawing | | C, CL, P+++ | 99.2 | 96.6 | 7.9 | 51.3 | 40.8 |
| | 40°C-2W | | C, WO, P+++ | 99.0 | 96.0 | 11.7 | 52.0 | 36.3 |
| | 40°C-1M | | C, WO, P+++ | 97.7 | 95.6 | 18.5 | 50.7 | 30.8 |
| F7 | T0 | 6.81 | C, CL, particle-free | 99.2 | 96.8 | 7.9 | 51.3 | 40.9 |
| | Shaking for 3D | | C, CL, particle-free | 99.3 | 96.9 | 7.9 | 51.8 | 40.4 |
| | 5 cycles of freeze-thawing | | C, CL, P+ | 99.2 | 96.8 | 7.9 | 51.6 | 40.5 |
| | 40°C-2W | | C, CL, particle-free | 98.9 | 96.4 | 13.4 | 51.4 | 35.3 |
| | 40°C-1M | | C, CL, P+ | 97.4 | 94.7 | 22.6 | 48.9 | 28.5 |
| F8 | T0 | 6.80 | C, CL, particle-free | 99.2 | 96.6 | 7.9 | 51.6 | 40.5 |
| | Shaking for 3D | | C, CL, particle-free | 99.2 | 97.0 | 8.0 | 51.6 | 40.5 |
| | 5 cycles of freeze-thawing | | C, CL, particle-free | 99.2 | 96.1 | 7.9 | 51.6 | 40.5 |
| | 40°C-2W | | C, CL, particle-free | 99.0 | 95.7 | 13.4 | 51.7 | 34.9 |
| | 40°C-1M | | C, CL, P | 97.6 | 94.8 | 22.8 | 49.2 | 28.1 |
| F10 | T0 | 6.98 | C, CL, particle-free | 99.2 | 96.4 | 7.4 | 53.2 | 39.5 |
| | Shaking for 3D | | C, CL, P+ | 99.2 | 96.8 | 8.1 | 51.4 | 40.5 |
| | 5 cycles of freeze-thawing | | C, CL, P+ | 99.2 | 95.6 | 7.4 | 53.3 | 39.4 |
| | 40°C-2W | | C, CL, P | 98.9 | 95.1 | 13.5 | 51.5 | 35.0 |
| | 40°C-1M | | C, CL, P+ | 98.5 | 94.6 | 21.7 | 49.4 | 29.0 |
| F11 | T0 | 7.37 | C, CL, particle-free | 99.3 | 95.7 | 7.9 | 51.5 | 40.6 |
| | Shaking for 3D | | C, SO, P+ | 99.2 | 96.9 | 8.1 | 51.5 | 40.3 |
| | 5 cycles of freeze-thawing | | C, WO, P+ | 99.2 | 96.5 | 7.5 | 53.3 | 39.2 |
| | 40°C-2W | | C, SO, P+++ | 98.9 | 95.7 | 15.4 | 51.1 | 33.5 |
| | 40°C-1M | | C, SO, P+++ | 98.6 | 94.9 | 25.3 | 48.5 | 26.2 |

| | | | | | | | | |
|---|---|---|---|---|---|---|---|---|
| Note: C = colorless; CL = clear; WO = micro-opalescent; SO = strongly opalescent; P (several particles); P+ (a small number of particles); P++ (many particles); P+++ (a large number of particles); The appearance results showed that: after 5 cycles of freeze-thawing, all formulas containing the 10 mM histidine-histidine hydrochloride pH 6.5 system showed generation of a large number of particles; formulas F4 and F6 also showed generation of a large number of particles after shaking for 3D, and F2, F3, and F5 didn't show generation of particles after shaking; the formulas containing the 10 mM histidine-histidine hydrochloride pH 7.0 system (F7 and F8) didn't show generation of particles after shaking for 3D, and after 5 cycles of freeze-thawing, only F7 showed generation of a small number of particles; under conditions of 40 °C-2W, except for F7 and F8, the remaining formulas all showed generation of particles, with a large number of particles generated in F3 (8% sucrose + 0.06% polysorbate 80), F4 (8% sucrose + 0.08% polysorbate 80), F5 (100 mM Gly + 4% sucrose + 0.04% polysorbate 80), and F6 (100 mM Pro + 4% sucrose + 0.04% polysorbate 80) and only a small number of particles generated in F2 (8% sucrose + 0.04% polysorbate 80); under conditions of 40 °C-1M, all formulas showed generation of particles, F7 and F8 still showed good performance with a small number of particles generated in F7 and several particles generated in F8, and the 10 mM histidine-histidine hydrochloride pH 7.0 system was significantly better than the 10 mM histidine-histidine hydrochloride pH 6.5 and 10 mM Tris-hydrochloric acid system; under conditions of 40 °C-1M, F2-F6 all showed generation of a large number of particles, indicating that the addition of glycine and proline and the increase of polysorbate 80 concentration did not significantly improve the appearance. | | | | | | | | |

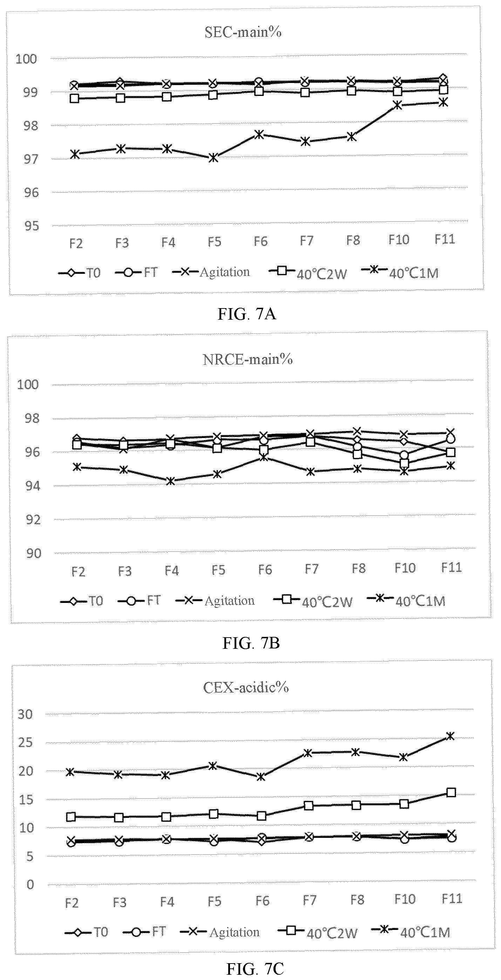

The SEC (FIG. 7A) results showed that: the SEC values under conditions of freeze-thawing and shaking showed no significant difference compared with T0; under conditions of high temperature, the SEC values of different formulas were reduced compared with T0; there was no significant difference between formulas F2-F8 (10 mM histidine-histidine hydrochloride system); the SEC purity of F10 and F11 (10 mM Tris-hydrochloric acid system) was slightly higher than that of the 10 mM histidine-histidine hydrochloride system.

The NRCE (FIG. 7B) results showed that: the NRCE values under conditions of freeze-thawing and shaking showed no significant difference compared with T0; under conditions of high temperature, the NRCE values of different formulas were reduced compared with T0, and there was no significant difference between formulas.

The CEX (FIG. 7C) results showed that: the results about acidic peak under conditions of freeze-thawing and shaking showed no significant difference compared with T0; under conditions of high temperature, the acid peaks of different formulas were increased compared with T0, and the acid peaks were significantly increased with the increase of pH in the same buffer system.

Taking the appearance, SEC, NRCE, and CEX results into consideration, the results of test items for purity showed slight changes, and the changes were all within acceptable ranges. In terms of appearance, the 10 mM histidine-histidine hydrochloride pH 7.0 system was better than the 10 mM histidine-histidine hydrochloride pH 6.5 and 10 mM Tris-hydrochloric acid system. According to the results of pH/buffer screening and auxiliary material and surfactant screening, the stability of the antibody was good when the pH was in the range of 6.5-7.5, the histidine-histidine hydrochloride system was better than the sodium dihydrogen phosphate-disodium hydrogen phosphate, Tris-hydrochloric acid, acetic acid-sodium acetate, Tris-citric acid, and other systems, and the 3882 antibody was sensitive to the ionic strength. The formulations were further optimized by using a histidine-antibody self-buffering system.

### Example 6. Screening for Buffer System and Surfactant Type of Anti-FXI/FXIa Antibody Formulation

The histidine-antibody self-buffering system pH 7.0 was selected to prepare anti-FXI/FXIa antibody formulations containing 60 mg/mL protein, 80 mg/mL sucrose as the auxiliary material, and poloxamer 188 or polysorbate 80 as the surfactant. The samples were placed in an incubator at 40±2 °C and 75±5% RH for stability examination:
F1) 35 mM histidine, pH 7.0, 1 mg/mL polysorbate 80, 80 mg/mL sucrose;
F2) 35 mM histidine, pH 7.0, 1 mg/mL poloxamer 188, 80 mg/mL sucrose;
F3) 35 mM histidine pH 7.0, 2 mg/mL poloxamer 188, 80 mg/mL sucrose;

**Table. 8. Confirmation of buffer system and screening for surfactant type**

| No. | Conditions | Actual pH | Appearance | SEC % | NRCE % | CEX % | | |
|---|---|---|---|---|---|---|---|---|
| | | | | Main peak | Main peak | Acidic peak | Main peak | Basic peak |
| F1 | T0 | 7.02 | C, CL, particle-free | 98.7 | 97.3 | 7.4 | 55.7 | 36.9 |
| | 40°C-2W | N/A | C, CL, particle-free | 96.9 | 94.9 | 20.0 | 50.1 | 29.9 |
| | 40°C-4W | N/A | Y+, CL, particle-free | 94.7 | 94.2 | 31.1 | 68.87(unable to be separated) | |
| F2 | T0 | 7.01 | C, CL, particle-free | 98.7 | 97.3 | 8.0 | 54.0 | 38.0 |
| | 40°C-2W | N/A | C, CL, particle-free | 98.1 | 96.7 | 16.7 | 52.8 | 30.6 |
| | 40°C-4W | N/A | Y, CL, particle-free | 97.4 | 93.3 | 26.1 | 49.8 | 24.2 |
| F3 | T0 | 7.00 | C, CL, particle-free | 98.7 | 97.2 | 7.9 | 53.9 | 38.2 |
| | 40°C-2W | N/A | C, CL, particle-free | 98.1 | 96.8 | 16.5 | 52.9 | 30.6 |
| | 40°C-4W | N/A | Y, CL, particle-free | 97.3 | 93.6 | 26.2 | 49.4 | 24.5 |

| | | | | | | | | |
|---|---|---|---|---|---|---|---|---|
| Note: C = colorless; Y = yellowish; Y+ = yellow; CL = clear | | | | | | | | |

The appearance results showed that all formulas showed no generation of particles under conditions of 40 °C-2W and 40 °C-4W.

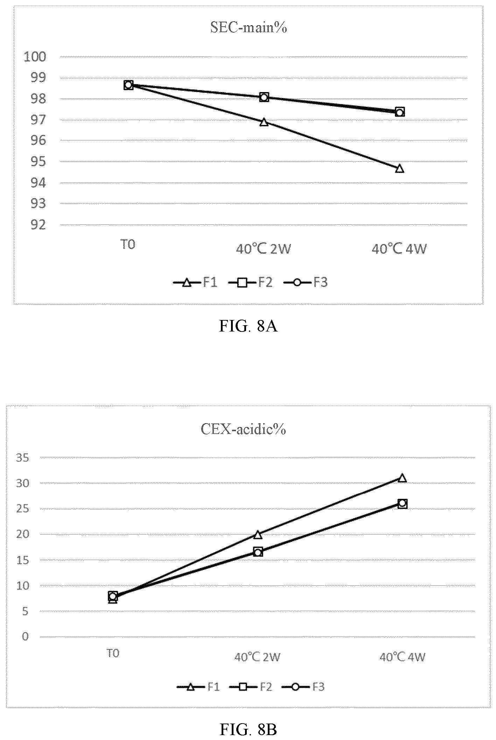

The SEC results (FIG. 8A) results showed that: under conditions of high temperature, the SEC values of different formulas were reduced compared with T0, with formula F1 (35 mM His + 8% sucrose + 0.1% polysorbate 80) reduced the most and significantly lower than F2 (35 mM His + 8% sucrose + 0.1% poloxamer 188) and F3 (35 mM His + 8% sucrose + 0.2% poloxamer 188).

The NRCE results showed that under conditions of high temperature, the NRCE values of different formulas were reduced compared with T0, and there was no significant difference between formulas. The CEX (FIG. 8B) results showed that: under conditions of high temperature, the acid peaks of different formulas were increased compared with T0, with the formula F1 increased the most and significantly higher than F2 and F3.

Taking the appearance, SEC, NRCE, and CEX results into consideration, when the histidine-antibody self-buffering system was used, the pH could be maintained around 7.0, and all formulas showed no generation of particles under conditions of 40 °C, and in the case of using polysorbate 80 as the surfactant, the results of formula purity test item were significantly different from T0. Thus, the pH/buffer was determined to be the histidine-antibody self-buffering system pH 7.0, and the surfactant was preferably poloxamer 188.

### Example 7. Confirmation of Buffer System and Screening for Surfactant Concentration for Anti-FXI/FXIa Antibody Formulation

Considering the final dose of the drug, the concentration of the anti-FXI/FXIa antibody was increased, and the histidine-antibody self-buffering system pH 7.0 was selected to prepare formulations containing 80 mg/mL protein, 80 mg/mL sucrose as the auxiliary material, and 1-2 mg/mL poloxamer 188 as the surfactant. The samples were placed under conditions of 5 cycles of -35 °C/room temperature freeze-thawing, 40 °C-2W, 40 °C-4W, and shaking for 3 days (25 °C, 300 rpm) for stability examination:
F1) 35 mM histidine pH 7.0, 1 mg/mL poloxamer 188, 80 mg/mL sucrose;
F2) 35 mM histidine pH 7.0, 1.5 mg/mL poloxamer 188, 80 mg/mL sucrose;
F3) 35 mM histidine pH 7.0, 2 mg/mL poloxamer 188, 80 mg/mL sucrose;

**Table 9. Results of confirmation of buffer system and screening for surfactant concentration for anti-FXI/FXIa antibody**

| No. | Conditions | Actual pH | Appearance | SEC % | NRCE % | CEX % | | |
|---|---|---|---|---|---|---|---|---|
| | | | | Main peak | Main peak | Acidic peak | Main peak | Basic peak |
| F1 | T0 | 7.00 | Y, WO, particle-free | 99.4 | 96.0 | 7.4 | 63.0 | 29.6 |
| | Shaking for 3D | N/A | Y, WO, particle-free | 99.6 | 96.1 | 7.8 | 63.7 | 28.5 |
| | 5 cycles of freeze-thawing | N/A | Y, WO, P+ | 99.6 | 96.3 | 7.6 | 63.4 | 29.1 |
| | 40°C-2W | 6.91 | Y, WO, particle-free | 98.7 | 95.5 | 17.4 | 60.6 | 22.1 |
| | 40°C-4W | 6.90 | Y, WO, P+ | 98.0 | 91.2 | 26.8 | 53.0 | 20.1 |
| F2 | T0 | 7.00 | Y, WO, particle-free | 99.4 | 96.1 | 7.5 | 62.6 | 29.9 |
| | Shaking for 3D | N/A | Y, WO, particle-free | 99.6 | 96.2 | 7.8 | 63.5 | 28.7 |
| | 5 cycles of freeze-thawing | N/A | Y, WO, P+ | 99.7 | 96.2 | 7.6 | 63.2 | 29.1 |
| | 40°C-2W | 6.93 | Y, WO, particle-free | 98.7 | 95.5 | 17.3 | 60.4 | 22.2 |
| | 40°C-4W | 6.90 | Y, WO, P+ | 97.9 | 90.6 | 27.0 | 52.9 | 20.1 |
| F3 | T0 | 7.00 | Y, WO, particle-free | 99.4 | 95.9 | 7.5 | 62.8 | 29.7 |
| | Shaking for 3D | N/A | Y, WO, particle-free | 99.6 | 96.2 | 7.7 | 63.9 | 28.4 |
| | 5 cycles of freeze-thawing | N/A | Y, WO, particle-free | 99.6 | 96.2 | 7.6 | 63.2 | 29.2 |
| | 40°C-2W | 6.93 | Y, WO, particle-free | 98.7 | 95.6 | 17.6 | 60.2 | 22.2 |
| | 40°C-4W | 6.88 | Y, WO, particle-free | 97.9 | 90.8 | 27.2 | 53.2 | 19.7 |

| | | | | | | | | |
|---|---|---|---|---|---|---|---|---|
| Note: Y = yellowish; WO = micro-opalescent; SO = strongly opalescent; P+ (a small number of particles) | | | | | | | | |

The appearance results showed that: all formulas showed no generation of particles after shaking for 3D; after 5 cycles of freeze-thawing, F1 (35 mM His, 0.1% poloxamer 188) and F2 (35 mM His, 0.15% poloxamer 188) showed generation of a small number of particles; under conditions of 40 °C-2W, all formulas showed no generation of particles; under conditions of 40 °C-4W, F1 and F2 showed generation of a small number of particles.

The SEC results showed that: the SEC values under conditions of freeze-thawing and shaking showed no significant difference compared with T0; under conditions of high temperature, the SEC values of different formulas were reduced compared with T0, and there was no significant difference between formulas.

The NRCE results showed that: the NRCE values under conditions of freeze-thawing and shaking showed no significant difference compared with T0; under conditions of high temperature, the NRCE values of different formulas were reduced compared with T0, and there was no significant difference between formulas.

The CEX results showed that: the results about acidic peak under conditions of freeze-thawing and shaking showed no significant difference compared with T0; under conditions of high temperature, the acid peaks of different formulas were increased compared with T0, and there was no significant difference between formulas.

Taking the appearance, SEC, NRCE, and CEX results into consideration, the pH/buffer was determined to be the histidine-antibody self-buffering system pH 7.0, and the surfactant was 0.2% poloxamer 188.

### Example 8. Screening for Antibody Concentrations in Anti-FXI/FXIa Antibody Formulation

The histidine-antibody self-buffering system pH 7.0 was selected to prepare formulations containing 60 mg/mL-100 mg/mL anti-FXI/FXIa antibody, 2 mg/mL poloxamer 188, and 80 mg/mL sucrose as the auxiliary material. The samples were placed under conditions of 5 cycles of -35 °C/room temperature freeze-thawing, 40 °C-2W, 40 °C-4W, and shaking for 3 days (25 °C, 300 rpm) for stability examination:
F1) 35 mM histidine pH 7.0, 2 mg/mL poloxamer 188, 80 mg/mL sucrose, 60 mg/mL protein;
F2) 35 mM histidine pH 7.0, 2 mg/mL poloxamer 188, 80 mg/mL sucrose, 80 mg/mL protein;
F3) 35 mM histidine pH 7.0, 2 mg/mL poloxamer 188, 80 mg/mL sucrose, 100 mg/mL protein;

**Table 10. Results of screening for protein concentration of anti-FXI/FXIa antibody formulation**

| No. | Conditions | Actual pH | Appearance | SEC % | NRCE % | CEX % | | |
|---|---|---|---|---|---|---|---|---|
| | | | | Main peak | Main peak | Acidic peak | Main peak | Basic peak |
| F1 | T0 | 7.17 | Y, WO, particle-free | 99.7 | 96.3 | 7.9 | 62.9 | 29.2 |
| | Shaking for 3D | N/A | Y, WO, particle-free | 99.8 | 96.5 | 7.9 | 61.6 | 30.4 |
| | 5 cycles of freeze-thawing | N/A | Y, WO, particle-free | 99.8 | 96.5 | 7.4 | 61.5 | 31.0 |
| | 40°C-2W | 7.06 | Y, WO, particle-free | 99.2 | 94.6 | 16.8 | 57.9 | 25.3 |
| | 40°C-4W | 7.11 | Y, WO, particle-free | 99.0 | 92.7 | 26.1 | 52.9 | 21.0 |
| F2 | T0 | 7.10 | Y, WO, particle-free | 99.7 | 96.2 | 8.0 | 62.7 | 29.4 |
| | Shaking for 3D | N/A | Y, WO, particle-free | 99.7 | 96.4 | 8.0 | 61.4 | 30.7 |
| | 5 cycles of freeze-thawing | N/A | Y, WO, particle-free | 99.8 | 96.5 | 7.8 | 61.3 | 30.9 |
| | 40°C-2W | 7.06 | Y, WO, particle-free | 99.0 | 94.8 | 16.8 | 58.0 | 25.2 |
| | 40°C-4W | 7.05 | Y, WO, particle-free | 98.3 | 93.4 | 25.5 | 53.2 | 21.3 |
| F3 | T0 | 7.00 | Y, WO, particle-free | 99.7 | 96.3 | 8.0 | 62.5 | 29.5 |
| | Shaking for 3D | N/A | Y, WO, particle-free | 99.7 | 96.5 | 7.9 | 61.5 | 30.6 |
| | 5 cycles of freeze-thawing | N/A | Y, WO, particle-free | 99.8 | 96.5 | 7.7 | 61.3 | 31.0 |
| | 40°C-2W | 7.00 | Y, WO, particle-free | 98.9 | 94.9 | 16.6 | 57.5 | 26.0 |
| | 40°C-4W | 6.98 | Y, WO, particle-free | 98.6 | 93.1 | 25.1 | 53.1 | 21.8 |

| | | | | | | | | |
|---|---|---|---|---|---|---|---|---|
| Note: Y = yellowish; WO = micro-opalescent | | | | | | | | |

The appearance results showed that all formulas showed no generation of particles under conditions of shaking for 3D, 5 cycles of freeze-thawing, 40 °C-2W and 40 °C-4W.

The SEC results showed that: the SEC values under conditions of freeze-thawing and shaking showed no significant difference compared with T0; under conditions of high temperature, the SEC values of different formulas were reduced compared with T0, and there was no significant difference between formulas.

The NRCE results showed that: the NRCE values under conditions of freeze-thawing and shaking showed no significant difference compared with T0; under conditions of high temperature, the NRCE values of different formulas were reduced compared with T0, and there was no significant difference between formulas.

The CEX results showed that: the results about acidic peak under conditions of freeze-thawing and shaking showed no significant difference compared with T0; under conditions of high temperature, the acid peaks of different formulas were increased compared with T0, and there was no significant difference between formulas.

Taking the appearance, SEC, NRCE, and CEX results into consideration, the formula was preliminarily determined as 35 mM histidine, 80 mg/mL sucrose, 2 mg/mL poloxamer 188, pH 7.0, and the concentration of the anti-FXI/FXIa antibody was 80 mg/mL.

### Example 9. Formula Stability Examination of Anti-FXI/FXIa Antibody Formulations

Considering the feasibility in production, the DoE design was carried out by using the histidine-protein self-buffering system and taking the concentration of histidines, the concentration of protein, and the concentration of poloxamer 188 as variables, and the factor levels were as follows: concentration of histidine, 30-40 mM; concentration of protein, 70-130 mg/mL; concentration of poloxamer 188, 1.8-2.8 g/L. DoE experiment was carried out by using the Minitab software to obtain a series of formulas. The samples were placed under conditions of 5 cycles of -35 °C/room temperature freeze-thawing, 40 °C-2W, 40 °C-26D, and shaking for 3 days (25 °C, 300 rpm) for stability examination:
F1) 30 mM histidine pH7.0, 1.8 mg/mL poloxamer 188, 80 mg/mL sucrose, 70 mg/mL protein;
F2) 30 mM histidine pH 7.0, 1.8 mg/mL poloxamer 188, 80 mg/mL sucrose, 130 mg/mL protein;
F3) 30 mM histidine pH 7.0, 2.8 mg/mL poloxamer 188, 80 mg/mL sucrose, 70 mg/mL protein;
F4) 30 mM histidine pH 7.0, 2.8 mg/mL poloxamer 188, 80 mg/mL sucrose, 130 mg/mL protein;
F5) 40 mM histidine pH 7.0, 1.8 mg/mL poloxamer 188, 80 mg/mL sucrose, 70 mg/mL protein;
F6) 40 mM histidine pH 7.0, 1.8 mg/mL poloxamer 188, 80 mg/mL sucrose, 130 mg/mL protein;
F7) 40 mM histidine pH 7.0, 2.8 mg/mL poloxamer 188, 80 mg/mL sucrose, 70 mg/mL protein;
F8) 40 mM histidine pH 7.0, 2.8 mg/mL poloxamer 188, 80 mg/mL sucrose, 130 mg/mL protein;
F9) 35 mM histidine pH 7.0, 2.3 mg/mL poloxamer 188, 80 mg/mL sucrose, 100 mg/mL protein;
F10) 35 mM histidine pH 7.0, 2.3 mg/mL poloxamer 188, 80 mg/mL sucrose, 100 mg/mL protein;
F11) 35 mM histidine pH 7.0, 2.3 mg/mL poloxamer 188, 80 mg/mL sucrose, 100 mg/mL protein;
F12) 35 mM histidine pH 7.0, 2 mg/mL poloxamer 188, 80 mg/mL sucrose, 120 mg/mL protein;

**Table 11. Results of formula stability of anti-FXI/FXIa antibody formulations**

| No. | Conditions | Actual pH | Appearance | SEC % | NRCE % | CEX % | | |
|---|---|---|---|---|---|---|---|---|
| | | | | Main peak | Main peak | Acidic peak | Main peak | Basic peak |
| F1 | T0 | 6.99 | C-Y, CL, particle-free | 99.3 | 97.1 | 8.0 | 60.7 | 31.2 |
| | Shaking for 3D | 7.00 | C-Y, CL, particle-free | 99.2 | 97.1 | 8.2 | 60.6 | 31.2 |
| | 5 cycles of freeze-thawing | 7.02 | C-Y, CL, particle-free | 99.2 | 97.5 | 7.8 | 61.8 | 30.4 |
| | 40°C-2W | 7.07 | C-Y, CL, particle-free | 99.3 | 95.8 | 16.6 | 58.0 | 25.4 |
| | 40°C-26D | 7.10 | C-Y, CL, particle-free | 99.0 | 93.1 | 24.5 | 53.6 | 22.0 |
| F2 | T0 | 6.85 | C-Y, CL, particle-free | 99.2 | 97.1 | 8.1 | 60.8 | 31.1 |
| | Shaking for 3D | 6.87 | C-Y, CL, particle-free | 99.2 | 97.0 | 8.2 | 60.8 | 31.0 |
| | 5 cycles of freeze-thawing | 6.89 | C-Y, CL, particle-free | 99.2 | 97.5 | 7.9 | 61.7 | 30.4 |
| | 40°C-2W | 6.94 | C-Y, CL, particle-free | 99.0 | 95.9 | 15.9 | 57.9 | 26.2 |
| | 40°C-26D | 6.94 | C-Y, CL, particle-free | 98.5 | 93.0 | 23.0 | 53.9 | 23.0 |
| F3 | T0 | 7.01 | C-Y, CL, particle-free | 99.2 | 97.1 | 8.1 | 60.8 | 31.1 |
| | Shaking for 3D | 7.01 | C-Y, CL, particle-free | 99.1 | 97.1 | 8.3 | 60.7 | 31.0 |
| | 5 cycles of freeze-thawing | 7.02 | C-Y, CL, particle-free | 99.2 | 97.5 | 7.9 | 61.3 | 30.8 |
| | 40°C-2W | 7.09 | C-Y, CL, particle-free | 99.3 | 96.3 | 16.8 | 57.9 | 25.2 |
| | 40°C-26D | 7.08 | C-Y, CL, particle-free | 99.0 | 93.2 | 24.5 | 53.6 | 21.9 |
| F4 | T0 | 6.86 | C-Y, CL, particle-free | 99.3 | 97.1 | 8.0 | 60.5 | 31.5 |
| | Shaking for 3D | 6.85 | C-Y, CL, particle-free | 99.0 | 97.1 | 8.3 | 60.8 | 31.0 |
| | 5 cycles of freeze-thawing | 6.85 | C-Y, CL, particle-free | 99.2 | 97.4 | 8.0 | 61.8 | 30.3 |
| | 40°C-2W | 6.94 | C-Y, CL, particle-free | 99.0 | 96.0 | 16.0 | 57.9 | 26.1 |
| | 40°C-26D | 6.94 | C-Y, CL, particle-free | 98.5 | 92.9 | 23.2 | 53.9 | 22.9 |
| F5 | T0 | 7.08 | C-Y, CL, particle-free | 99.3 | 97.1 | 8.1 | 60.6 | 31.2 |
| | Shaking for 3D | 7.08 | C-Y, CL, particle-free | 99.2 | 97.1 | 8.4 | 60.7 | 30.9 |
| | 5 cycles of freeze-thawing | 7.07 | C-Y, CL, particle-free | 99.2 | 97.3 | 7.7 | 61.6 | 30.7 |
| | 40°C-2W | 7.17 | C-Y, CL, particle-free | 99.4 | 96.1 | 17.7 | 57.5 | 24.8 |
| | 40°C-26D | 7.12 | C-Y, CL, particle-free | 99.0 | 92.9 | 25.9 | 53.0 | 21.1 |
| F6 | T0 | 6.93 | C-Y, CL, particle-free | 99.2 | 97.3 | 8.1 | 60.6 | 31.3 |
| | Shaking for 3D | 6.92 | C-Y, CL, particle-free | 99.2 | 97.0 | 8.3 | 60.8 | 31.0 |
| | 5 cycles of freeze-thawing | 6.94 | C-Y, CL, particle-free | 99.2 | 97.0 | 8.0 | 61.3 | 30.6 |
| | 40°C-2W | 7.01 | C-Y, CL, particle-free | 99.0 | 95.4 | 16.9 | 57.5 | 25.6 |
| | 40°C-26D | 6.96 | C-Y, CL, particle-free | 98.6 | 92.7 | 24.7 | 53.3 | 22.0 |
| F7 | T0 | 7.08 | C-Y, CL, particle-free | 99.2 | 97.1 | 8.1 | 60.7 | 31.2 |
| | Shaking for 3D | 7.08 | C-Y, CL, particle-free | 99.2 | 97.0 | 8.4 | 60.3 | 31.4 |
| | 5 cycles of freeze-thawing | 7.08 | C-Y, CL, particle-free | 99.2 | 97.1 | 7.8 | 61.5 | 30.7 |
| | 40°C-2W | 7.15 | C-Y, CL, particle-free | 99.4 | 95.9 | 17.7 | 57.7 | 24.6 |
| | 40°C-26D | 7.11 | C-Y, CL, particle-free | 99.0 | 92.8 | 26.1 | 52.9 | 21.0 |
| F8 | T0 | 6.91 | C-Y, CL, particle-free | 99.2 | 97.2 | 8.1 | 60.8 | 31.1 |
| | Shaking for 3D | 6.94 | C-Y, CL, particle-free | 99.0 | 97.0 | 8.3 | 60.8 | 30.9 |
| | 5 cycles of freeze-thawing | 6.94 | C-Y, CL, particle-free | 99.3 | 97.0 | 8.1 | 61.8 | 30.1 |
| | 40°C-2W | 7.04 | C-Y, CL, particle-free | 99.1 | 95.7 | 16.8 | 57.7 | 25.5 |
| | 40°C-26D | 7.00 | C-Y, CL, particle-free | 98.6 | 92.8 | 24.5 | 53.4 | 22.1 |
| F9 | T0 | 6.95 | C-Y, CL, particle-free | 99.3 | 97.1 | 8.1 | 60.4 | 31.5 |
| | Shaking for 3D | 6.96 | C-Y, CL, particle-free | 99.2 | 97.1 | 8.3 | 60.9 | 30.8 |
| | 5 cycles of freeze-thawing | 6.97 | C-Y, CL, particle-free | 99.2 | 97.1 | 8.1 | 61.7 | 30.3 |
| | 40°C-2W | 7.04 | C-Y, CL, particle-free | 99.1 | 95.9 | 17.1 | 57.9 | 25.1 |
| | 40°C-26D | 7.02 | C-Y, CL, particle-free | 98.7 | 92.6 | 24.9 | 53.2 | 21.8 |
| F10 | T0 | 6.99 | C-Y, CL, particle-free | 99.3 | 97.1 | 8.2 | 60.7 | 31.2 |
| | Shaking for 3D | 6.95 | C-Y, CL, particle-free | 99.0 | 97.0 | 8.3 | 60.8 | 30.9 |
| | 5 cycles of freeze-thawing | 6.97 | C-Y, CL, particle-free | 99.2 | 97.1 | 8.0 | 61.6 | 30.4 |
| | 40°C-2W | 7.09 | C-Y, CL, particle-free | 99.1 | 95.8 | 17.0 | 57.6 | 25.4 |
| | 40°C-26D | 7.02 | C-Y, CL, particle-free | 98.7 | 92.6 | 24.7 | 53.4 | 21.9 |
| F11 | T0 | 6.99 | C-Y, CL, particle-free | 99.2 | 97.1 | 8.1 | 60.7 | 31.2 |
| | Shaking for 3D | 6.97 | C-Y, CL, particle-free | 99.2 | 97.0 | 8.2 | 60.8 | 31.0 |
| | 5 cycles of freeze-thawing | 6.97 | C-Y, CL, particle-free | 99.2 | 96.9 | 8.1 | 61.5 | 30.4 |
| | 40°C-2W | 7.04 | C-Y, CL, particle-free | 99.1 | 95.9 | 16.8 | 57.9 | 25.3 |
| | 40°C-26D | 7.03 | C-Y, CL, particle-free | 98.7 | 92.7 | 24.6 | 53.5 | 21.9 |
| F12 | T0 | 6.89 | C-Y, CL, particle-free | 99.8 | 97.2 | 7.6 | 61.1 | 31.4 |
| | Shaking for 3D | 6.99 | C-Y, CL, particle-free | 99.0 | 97.4 | 7.8 | 61.6 | 30.6 |
| | 5 cycles of freeze-thawing | 7.01 | C-Y, CL, particle-free | 99.8 | 98.2 | 7.7 | 61.0 | 31.4 |
| | 40°C-2W | 6.97 | C-Y, CL, particle-free | 99.4 | 96.8 | 16.1 | 58.8 | 25.2 |
| | 40°C-4W | 6.94 | C-Y, CL, particle-free | 98.2 | 95.9 | 23.7 | 54.4 | 21.9 |

| | | | | | | | | |
|---|---|---|---|---|---|---|---|---|
| Note: C = colorless; Y = yellowish; CL = clear | | | | | | | | |

The appearance results showed that all formulas showed no generation of particles under conditions of shaking for 3D, 5 cycles of freeze-thawing, 40 °C-2W, and 40 °C-26D.

The SEC results showed that: the SEC values under conditions of freeze-thawing and shaking showed no significant difference compared with T0; under conditions of high temperature, the SEC values of different formulas were reduced compared with T0, and there was no significant difference between formulas.

The NRCE results showed that: the NRCE values under conditions of freeze-thawing and shaking showed no significant difference compared with T0; under conditions of high temperature, the NRCE values of different formulas were reduced compared with T0, and there was no significant difference between formulas.

The CEX results showed that: the results about acidic peak under conditions of freeze-thawing and shaking showed no significant difference compared with T0; under conditions of high temperature, the acid peaks of different formulas were increased compared with T0, and there was no significant difference between formulas.

Taking the appearance, SEC, NRCE, and CEX results into consideration, when the concentration of the anti-FXI/FXIa antibody was 70-130 mg/mL, the concentration of histidine was 30-40 mM, and the concentration of poloxamer 188 was 1.8-2.8 g/L, the results of the purity item showed no significant difference and the formulation showed good stability.

### Example 10. Stability of Anti-FXI Antibody Formulations in Use

The anti-FXI antibody formulation finished product was diluted in infusion bags separately with 0.9% normal saline injection (infusion bag material: polypropylene) and 5% glucose injection (infusion bag material: polypropylene) to 0.20 mg/mL and 13.30 mg/mL, respectively, and the stability of the diluted drug liquid in a disposable infusion tube (tube material: polypropylene; filter membrane material: polyether sulfone) under conditions of 2-8 °C for 24 h + illumination at room temperature for 6 h. The results of the experiment are shown in Table 12.

After the anti-FXI antibody formulation was diluted with 5% glucose and 0.9% normal saline (0.20-13.30 mg/mL) and then subjected to conditions of 2-8 °C for 24 h + illumination at room temperature for 6 h, all the test items for examination were within the range of acceptable criteria. Therefore, the diluted drug liquid showed good compatibility with the infusion system, and the formulation could be used within 24 h at 2-8 °C and 6 h at room temperature after being diluted in the infusion system.

**Table 12. Stability results of anti-FXI antibody formulation in use**

| Diluting medium | Concentration | Conditions | Appearance | Protein content (mg/mL) | Insoluble microparticle | | SEC (%) | NRCE (%) | CEX (%) | Binding activity (%) | Bacterial endotoxin (EU/mg) |
|---|---|---|---|---|---|---|---|---|---|---|---|
| | | | | | ≥ 10 µm | ≥ 25 µm | | | Main peak | | |
| Acceptance criteria | | | C-Y, CL, particle-free | Target content±10% | ≥ 10 µm:≤ 6000/vial | ≥ 25 µm:≤ 600/vial | ≥95% | ≥90% | ≥50% | Reference substance activity ±30% | < 0.25 EU/mg |
| 0.9% normal saline | 0.20 mg/mL | 0h | C-Y, CL, particle-free | 0.21 | 100 | 100 | 99.8 | 96.8 | 62.5 | 86% | N/A |
| | | 24h±6h | C-Y, CL, particle-free | 0.22 | 100 | 100 | 99.7 | 96.6 | 62.4 | 105% | Meets the criteria |
| | 13.30 mg/mL | 0h | C-Y, CL, particle-free | 13.94 | 200 | 100 | 99.8 | 97.0 | 62.5 | 86% | N/A |
| | | 24h±6h | C-Y, CL, particle-free | 13.80 | 200 | 100 | 99.7 | 97.1 | 62.6 | 85% | Meets the criteria |
| 5% glucose | 0.20 mg/mL | 0h | C-Y, CL, particle-free | 0.22 | 100 | 100 | 99.8 | 97.5 | 61.9 | 83% | N/A |
| | | 24h±6h | C-Y, CL, particle-free | 0.22 | 200 | 100 | 99.8 | 97.1 | 62.0 | 95% | Meets the criteria |
| | 13.30 mg/mL | 0h | C-Y, CL, particle-free | 13.97 | 200 | 100 | 99.7 | 97.3 | 62.5 | 83% | N/A |
| | | 24h±6h | C-Y, CL, particle-free | 1406 | 100 | 100 | 99.7 | 97.0 | 62.7 | 99% | Meets the criteria |

| | | | | | | | | | | | |
|---|---|---|---|---|---|---|---|---|---|---|---|
| Note: C = colorless; Y = yellowish, CL = clear; | | | | | | | | | | | |

## Claims

1. A pharmaceutical composition, comprising an anti-FXI/FXIa antibody or an antigen-binding fragment thereof and a buffer selected from the group consisting of one or more of an acetate buffer, a histidine salt buffer, a Tris-hydrochloride buffer, a Tris-citrate buffer, and a phosphate buffer, wherein
preferably, the buffer is a histidine salt buffer, a Tris-hydrochloride buffer, or a phosphate buffer.

2. The pharmaceutical composition according to claim 1, wherein the buffer or the pharmaceutical composition has a pH of about 4.0 to about 8.5, preferably about 6.5 to about 7.5, and more preferably about 7.0.

3. The pharmaceutical composition according to claim 1 or 2, wherein the buffer has a concentration of about 5 mM to about 100 mM, preferably about 10 mM to about 30 mM, and more preferably about 5 mM to about 15 mM.

4. The pharmaceutical composition according to any one of claims 1 to 3, wherein the anti-FXI/FXIa antibody or the antigen-binding fragment thereof has a concentration of about 1 mg/mL to about 300 mg/mL and preferably about 50 mg/mL to about 150 mg/mL.

5. The pharmaceutical composition according to any one of claims 1 to 4, further comprising a surfactant, wherein
preferably, the surfactant is poloxamer 188, polysorbate 80, or polysorbate 20.

6. The pharmaceutical composition according to claim 5, wherein the surfactant has a concentration of about 0.1 mg/mL to about 10 mg/mL, preferably about 1.5 mg/mL to about 4 mg/mL, and preferably about 0.4 mg/mL to about 0.8 mg/mL.

7. The pharmaceutical composition according to any one of claims 1 to 6, further comprising an osmotic pressure regulator, wherein
preferably, the osmotic pressure regulator is selected from the group consisting of one or more of the group consisting of sucrose, trehalose, sorbitol, arginine, proline, glycine, and sodium chloride; more preferably, the osmotic pressure regulator is selected from the group consisting of sucrose, proline, and sucrose, or glycine and sucrose.

8. The pharmaceutical composition according to claim 7, wherein the osmotic pressure regulator is selected from the group consisting of:
about 10 mg/mL to about 150 mg/mL sucrose,
about 10 mM to 200 mM glycine and about 10 mg/mL to about 100 mg/mL sucrose, and
about 10 mM to 200 mM proline and about 10 mg/mL to about 100 mg/mL sucrose;
preferably, the osmotic pressure regulator is selected from the group consisting of:
about 80 mg/mL sucrose,
about 100 mM glycine and about 40 mg/mL sucrose, and
about 100 mM proline and about 40 mg/mL sucrose.

9. A pharmaceutical composition, comprising an anti-FXI/FXIa antibody or an antigen-binding fragment thereof, wherein the pharmaceutical composition is a self-buffering system; preferably, the pharmaceutical composition comprises a basic amino acid; more preferably, the basic amino acid is histidine.

10. The pharmaceutical composition according to claim 9, wherein the anti-FXI/FXIa antibody or the antigen-binding fragment thereof has a concentration of about 1 mg/mL to about 300 mg/mL, preferably about 30 mg/mL to about 200 mg/mL, and more preferably about 70 mg/mL to about 130 mg/mL.

11. The pharmaceutical composition according to claim 9 or 10, wherein the basic amino acid has a concentration of about 1 mM to about 100 mM, preferably 10 mM to about 60 mM, more preferably about 30 mM to about 40 mM, and most preferably about 35 mM.

12. The pharmaceutical composition according to any one of claims 9 to 11, wherein the pharmaceutical composition has a pH of about 4.0 to about 8.5, preferably about 6.5 to about 7.5, more preferably about 6.7 to about 7.3, and most preferably about 7.0.

13. The pharmaceutical composition according to any one of claims 9 to 12, further comprising an osmotic pressure regulator, wherein
preferably, the osmotic pressure regulator is selected from the group consisting of one or more of sucrose, trehalose, sorbitol, arginine, proline, glycine, and sodium chloride;
more preferably, the osmotic pressure regulator is sucrose.

14. The pharmaceutical composition according to claim 13, wherein the osmotic pressure regulator has a concentration of about 10 mg/mL to about 400 mg/mL, preferably about 50 mg/mL to about 200 mg/mL, and more preferably about 80 mg/mL.

15. The pharmaceutical composition according to any one of claims 9 to 14, further comprising a surfactant, wherein
preferably, the surfactant is poloxamer 188, polysorbate 80, or polysorbate 20.

16. The pharmaceutical composition according to claim 15, wherein the surfactant has a concentration of about 0.1 mg/mL to about 10 mg/mL, preferably about 1.8 mg/mL to about 2.8 mg/mL, and more preferably about 2 mg/mL.

17. A pharmaceutical composition, comprising (a) and (b), and optionally, further comprising (c) and/or (d), wherein
(a) about 1 mg/mL to about 500 mg/mL anti-FXI/FXIa antibody,
(b) about 1 mM to about 100 mM histidine,
(c) about 10 mg/mL to about 400 mg/mL sucrose or trehalose, and
(d) about 0.5 mg/mL to about 80 mg/mL poloxamer, polysorbate 80, or polysorbate 20,
the pharmaceutical composition having a pH of about 4.0 to about 8.5;
or,
(a) about 30 mg/mL to about 200 mg/mL anti-FXI/FXIa antibody,
(b) about 10 mM to about 60 mM histidine,
(c) about 50 mg/mL to about 200 mg/mL sucrose, and
(d) about 1.5 mg/mL to about 5.0 mg/mL poloxamer 188,
the pharmaceutical composition having a pH of about 6.5 to about 7.5;
or,
(a) about 70 mg/mL to about 130 mg/mL anti-FXI/FXIa antibody,
(b) about 30 mM to about 40 mM histidine,
(c) about 60 mg/mL to about 150 mg/mL sucrose, and
(d) about 1.8 mg/mL to about 2.8 mg/mL poloxamer 188,
the pharmaceutical composition having a pH of about 6.7 to about 7.3;
or,
(a) about 70 mg/mL to about 200 mg/mL anti-FXI/FXIa antibody,
(b) about 30 mM to about 100 mM histidine,
(c) about 60 mg/mL to about 150 mg/mL sucrose, and
(d) about 1.8 mg/mL to about 2.8 mg/mL poloxamer 188,
the pharmaceutical composition having a pH of about 6.7 to about 7.3.

18. The pharmaceutical composition according to claim 17, comprising:
(a) about 70 mg/mL to about 130 mg/mL anti-FXI/FXIa antibody or antigen-binding fragment thereof,
(b) about 35 mM histidine,
(c) about 80 mg/mL sucrose, and
(d) about 2 mg/mL poloxamer 188,
the pharmaceutical composition having a pH of about 7.0; or
(a) about 70 mg/mL to about 130 mg/mL anti-FXI/FXIa antibody or antigen-binding fragment thereof,
(b) about 35 mM histidine,
(c) about 80 mg/mL sucrose, and
(d) about 2 mg/mL poloxamer 188,
the pharmaceutical composition having a pH of about 6.7; or
(a) about 70 mg/mL to about 130 mg/mL anti-FXI/FXIa antibody or antigen-binding fragment thereof,
(b) about 35 mM histidine,
(c) about 80 mg/mL sucrose, and
(d) about 2 mg/mL poloxamer 188,
the pharmaceutical composition having a pH of about 7.3; or
(a) about 70 mg/mL to about 130 mg/mL anti-FXI/FXIa antibody or antigen-binding fragment thereof,
(b) about 30 mM histidine,
(c) about 80 mg/mL sucrose, and
(d) about 1.8 mg/mL poloxamer 188,
the pharmaceutical composition having a pH of about 7.0; or
(a) about 70 mg/mL to about 130 mg/mL anti-FXI/FXIa antibody or antigen-binding fragment thereof,
(b) about 30 mM histidine,
(c) about 80 mg/mL sucrose, and
(d) about 2.8 mg/mL poloxamer 188,
the pharmaceutical composition having a pH of about 7.0; or
(a) about 70 mg/mL to about 130 mg/mL anti-FXI/FXIa antibody or antigen-binding fragment thereof,
(b) about 40 mM histidine,
(c) about 80 mg/mL sucrose, and
(d) about 1.8 mg/mL poloxamer 188,
the pharmaceutical composition having a pH of about 7.0; or
(a) about 70 mg/mL to about 130 mg/mL anti-FXI/FXIa antibody or antigen-binding fragment thereof,
(b) about 40 mM histidine,
(c) about 80 mg/mL sucrose, and
(d) about 2.3 mg/mL poloxamer 188,
the pharmaceutical composition having a pH of about 7.0; or
(a) about 70 mg/mL to about 130 mg/mL anti-FXI/FXIa antibody or antigen-binding fragment thereof,
(b) about 40 mM histidine,
(c) about 80 mg/mL sucrose, and
(d) about 2.8 mg/mL poloxamer 188,
the pharmaceutical composition having a pH of about 7.0; or
(a) about 70 mg/mL to about 130 mg/mL anti-FXI/FXIa antibody or antigen-binding fragment thereof,
(b) about 35 mM histidine,
(c) about 80 mg/mL sucrose, and
(d) about 2.3 mg/mL poloxamer 188,
the pharmaceutical composition having a pH of about 7.0; or
(a) about 70 mg/mL to about 130 mg/mL anti-FXI/FXIa antibody or antigen-binding fragment thereof,
(b) about 35 mM histidine,
(c) about 80 mg/mL sucrose, and
(d) about 1.8 mg/mL poloxamer 188,
the pharmaceutical composition having a pH of about 7.0.

19. A pharmaceutical composition, obtained by diluting the pharmaceutical composition according to claim 17 or 18 with 0.9% normal saline or 5% glucose solution or comprising an anti-FXI/FXIa antibody or an antigen-binding fragment thereof, histidine, sucrose, and poloxamer 188 each at a concentration suitable for intravenous injection obtained after diluting the pharmaceutical composition according to claim 16 with 0.9% normal saline or 5% glucose solution, wherein preferably, the anti-FXI/FXIa antibody or the antigen-binding fragment thereof has a concentration of about 0.01 mg/mL to about 50 mg/mL,
more preferably, the anti-FXI/FXIa antibody or the antigen-binding fragment thereof has a concentration of about 0.1 mg/mL to about 30 mg/mL, and
most preferably, the anti-FXI/FXIa antibody or the antigen-binding fragment thereof has a concentration of about 0.20 mg/mL to about 13.30 mg/mL.

20. The pharmaceutical composition according to any one of claims 1 to 19, wherein the anti-FXI/FXIa antibody or the antigen-binding fragment thereof comprises a heavy chain variable region and a light chain variable region, wherein the heavy chain variable region comprises a HCDR1, a HCDR2, and a HCDR3 set forth in SEQ ID NOs: 7, 8, and 9, respectively, and the light chain variable region comprises a LCDR1, a LCDR2, and a LCDR3 set forth in SEQ ID NOs: 10, 11, and 12, respectively.

21. The pharmaceutical composition according to any one of claims 1 to 20, wherein the anti-FXI/FXIa antibody or the antigen-binding fragment thereof comprises a heavy chain variable region and a light chain variable region, wherein the heavy chain variable region and the light chain variable region are selected from the group consisting of:
a heavy chain variable region whose sequence is set forth in SEQ ID NO: 5 or a sequence having at least 90% identity thereto and a light chain variable region whose sequence is set forth in SEQ ID NO: 6 or a sequence having at least 90% identity thereto;
a heavy chain variable region whose sequence is set forth in SEQ ID NO: 13 or a sequence having at least 90% identity thereto and a light chain variable region whose sequence is set forth in SEQ ID NO: 14 or a sequence having at least 90% identity thereto;
a heavy chain variable region whose sequence is set forth in SEQ ID NO: 15 or a sequence having at least 90% identity thereto and a light chain variable region whose sequence is set forth in SEQ ID NO: 16 or a sequence having at least 90% identity thereto; or
a heavy chain variable region whose sequence is set forth in SEQ ID NO: 17 or a sequence having at least 90% identity thereto and a light chain variable region whose sequence is set forth in SEQ ID NO: 16 or a sequence having at least 90% identity thereto.

22. The pharmaceutical composition according to any one of claims 1 to 21, wherein the anti-FXI/FXIa antibody or the antigen-binding fragment thereof further comprises a human IgG1 Fc or a human IgG4 Fc; preferably, the human IgG4 Fc comprises S241P mutation.

23. The pharmaceutical composition according to any one of claims 1 to 22, wherein the anti-FXI/FXIa antibody or the antigen-binding fragment thereof comprises a heavy chain and a light chain, wherein the heavy chain comprises the sequence set forth in SEQ ID NO: 21 or a sequence having at least 90% identity thereto, and the light chain comprises the sequence set forth in SEQ ID NO: 22 or a sequence having at least 90% identity thereto.

24. A lyophilized formulation, wherein the lyophilized formulation is obtained by lyophilizing the pharmaceutical composition according to any one of claims 1 to 23, or the lyophilized formulation, after being reconstituted, is capable of forming the pharmaceutical composition according to any one of claims 1 to 23.

25. A reconstituted solution, being prepared by reconstituting the lyophilized formulation according to claim 24.

26. The pharmaceutical composition according to any one of claims 1 to 23 or the reconstituted solution according to claim 25, being an intravenous injection, a subcutaneous injection, an intraperitoneal injection, or an intramuscular injection, and preferably an intravenous injection or a subcutaneous injection.

27. An article of manufacture, comprising a container, wherein the container contains the pharmaceutical composition according to any one of claims 1 to 23, the lyophilized formulation according to claim 24, or the reconstituted solution according to claim 25.

28. A method for treating or preventing a disease, comprising administering to a subject in need thereof a therapeutically or prophylactically effective amount of the pharmaceutical composition according to any one of claims 1 to 22, the lyophilized formulation according to claim 23, the reconstituted solution according to claim 25, or the article of manufacture according to claim 27, wherein
the disease is a thrombotic or thromboembolic disease and/or a thrombotic or thromboembolic complication, cardiac arrhythmia, cardiogenic thromboembolism, or disseminated intravascular coagulation;
preferably, the thrombotic or thromboembolic disease or the complication thereof is selected from the group consisting of: cardiac coronary artery diseases, such as acute coronary syndrome (ACS), ST-elevation myocardial infarction (STEMI), non-ST-elevation myocardial infarction (non-STEMI), stable angina pectoris, unstable angina pectoris, and reocclusion and restenosis after coronary interventions, thrombotic or thromboembolic diseases leading to peripheral arterial occlusive disease, pulmonary embolism, venous thromboembolism, venous thrombosis, transient ischemic attack, thrombotic stroke, and thromboembolic stroke in other blood vessels, pulmonary disease and pulmonary hypertension caused by chronic thromboembolism (CTEPH), and a combination thereof; preferably, the administration is performed by subcutaneous or intravenous injection.
